# EUROPEAN PATENT SPECIFICATION

(11) **EP 2 154 966 B1**
(45) Date of publication and mention of the grant of the patent: **21.08.2013**
(21) Application number: 08799882.9
(22) Date of filing: 21.04.2008
(51) Int. Cl.: C07D 235/08, C07D 235/18, C07D 405/04, C07D 405/12, A61K 31/4184, A61P 31/04, A61P 31/08

(54) **BENZIMIDAZOLES AND PHARMACEUTICAL COMPOSITIONS THEREOF**
BENZIMIDAZOLE UND PHARMAZEUTISCHE ZUSAMMENSETZUNGEN DARAUS
BENZIMIDAZOLES ET COMPOSITIONS PHARMACEUTIQUES DE CEUX-CI

(30) Priority: 20.04.2007 US 912980 P
(43) Date of publication of application: 24.02.2010
(73) Proprietor: The Research Foundation Of State University Of New York, Albany, NJ 12201-0009 (US)
(72) Inventor: OJIMA, Iwao, Port Jefferson, NY 11777-1462 (US); LEE, Seung-yub, Princeton, NJ 08540 (US)
(74) Representative: Hatzmann, Martin
(86) International application number: PCT/US2008/005084
(87) International publication number: WO 2008/130669

(56) References cited:
- WO-A1-2007/105023
- US-A1- 2005 124 678
- US-A1- 2006 116 412
- KYM, O. ET AL: "Substituted .alpha.-Hydroxy-and .alpha.-Methylbenzimidazoles" BERICHTE DER DEUTSCHEN CHEMISCHEN GESELLSCHAFT , 45, 3238-55 CODEN: BDCGAS; ISSN: 0365-9496, 1913, XP008128623

## Description

### BACKGROUND OF THE INVENTION

Tuberculosis (TB) was one of the first infectious diseases to be identified. More than fifty years of research has been directed to controlling and eliminating this disease. However, the eradication of TB is still one of the most prominent challenges for basic and clinical research scientists.

Once thought to be under control, TB case reports in the U.S. increased sharply in the early 1990's. Although, this trend has reversed and the reported numbers of new cases has steadily declined in industrialized countries, TB remains a major global public health threat. Recent statistics from the WHO estimate that there are approximately 8.4 million new cases every year with a global mortality rate of 23% or approximately 2 million deaths per year.

Poor chemotherapeutics and inadequate local-control programs contribute to the inability to manage TB and lead to the emergence of drug resistant strains of the bacteria that cause *Mycobacterium tuberculosis* (*Mtb*). A survey conducted at 58 international sites between 1996 and 1999 found exceptionally high rates of single and multidrug-resistant strains in Estonia, Latvia and Russia, and revealed that countries such as China and Iran were developing a high prevalence of multidrug-resistance (MDR-TB). See Kruuner, A., Sillastu, H., Danilovitsh, M., Levina, K., Svenson, S. B., Kallenius, G., and Hofrner, S. E. (1998) Drug resistant tuberculosis in Estonia, Int J Tuberc Lung Dis 1, 130-3. Significantly, MDR-TB is much more difficult to treat than sensitive TB, requiring administration of more expensive, second-line antibiotics for up to two years. The frequency of resistance to at least one of the first-line TB drugs (isoniazid (INH), rifampicin (RIF), pyrazinamide or ethambutol) ranged from 1.7% in Uruguay to 36.9% in Estonia. The frequency of resistance is indicative of the global problem involving not only the spread of *Mtb,* but also treatment.

Finally, of critical importance is the role of TB as a major opportunistic pathogen in patients with HIV/AIDS. Consequently, there is a pressing need for the development of novel TB drugs that are effective against both sensitive and resistant *Mtb* strains.

Likewise, new drugs are needed to treat patients infected by *Franciselia tulerensis,* the bacteria which causes tularemia. Tularemia is primarily enzootic, however, in humans, it causes lesions and flu-like symptoms. Finding new methods of treating *F*. *tulerensis* is of great importance because it is one of the most pathogenic microorganisms presently known. As such, it is currently listed as a category A select agent by the Centers for Disease Control and Prevention because of its potential as a bioterrorism agent.

US 2005/0124678 discloses substituted benzimidazole compounds useful as antiinfectives that decrease resistance, virulence, or growth of microbes. Methods of making and using the substituted benzimidazoles are described, as well as pharmaceutical preparations thereof, in, e.g. reducing antibiotic resistance and inhibiting biofilms.

WO 2007/105023 and the corresponding European patent application, EP-A-2008210, which is comprised in the state of the art pursuant to Article 54(3) EPC in relation to claims 1-10 appended hereto, discloses benzimidazole compounds which are active as inhibitors of glutamine synthetase. The compounds are indicated to be useful for the treatment of bacterial infections, including *Mycobacterium tuberculosis.*

### SUMMARY OF THE INVENI10N

One aspect of the invention relates to a molecule having formula I: 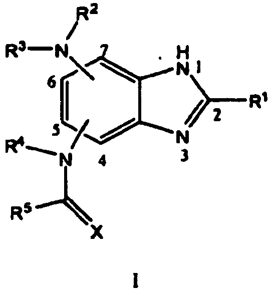 wherein:
R¹ represents NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶. CH₂OH, CR⁷R⁸OH. CH₂OR⁶, CR⁷R⁸OR⁶, CH₂NH₂, CR⁷R⁸NH₂, CR⁷R⁸NR⁹R¹⁰, alkyl, cycloalkyl, aryl, or halo;
R² and R⁴ independently represent H, alkyl, cycloalkyl, or aryl;
R³ represents alkyl, cycloalkyl, or aryl;
R⁵ represents H, R⁶, OR⁶, SR⁶, NH₂, NHR⁶, or NR⁹R¹⁰;
X represents O S, NH, or NR⁶;
R⁶, R⁷, R⁸, R⁹, and R¹⁰ independently represent alkyl, cycloalkyl, aryl, or halo;
R² and R³; R⁴ and R⁵; and R⁹ and R¹⁰ independently, may be combined to represent a heterocyclic alkyl or heterocyclic aryl;
R⁷ and R⁸ may be combined to represent a cycloalkyl;
alkyl groups are branched or unbranched, saturated or unsaturated, and have 1-18 carbon atoms in their longest chain;
cycloalkyl groups are carbocyclic or heterocyclic, fused or unfused, non-aromatic ring systems having a total of 5-16 ring members including substituent rings;
aryl groups are carbocyclic or heterocyclic;
carbocyclic aryl groups are fused or unfused ring systems having a total of 6-16 ring members including substituent rings;
heterocyclic aryl groups are fused or unfused ring systems having a total of 5-16 ring members including substituent rings;
halo substituents are fluoro, chloro, or bromo;
each alkyl, cycloalkyl, and aryl, independently, may be unsubstituted or substituted with one or more substituent at any position;
alkyl substituents are halo, hydroxyl, OR⁶, SR⁶, NH₂, NHR⁶, NR⁹R¹⁰, cycloalkyl, or aryl;
cycloalkyl substituents are halo, hydroxyl, OR⁶, SR⁶, NH₂, NHR⁶, NR⁹R¹⁰, alkyl, cycloalkyl, or aryl;
aryl substituents are halo, hydroxyl, OR⁶, SR⁶, NH₂, NHR⁶, NR⁹R¹⁰, alkyl, cycloalkyl, aryl, nitro, or carboxyl; and heterocyclic alkyl and heterocyclic aryl have at least one heteroatom selected from oxygen, nitrogen and sulfur; and
pharmaceutically acceptable salts thereof.

A further aspect of the invention relates to a molecule having the formula I 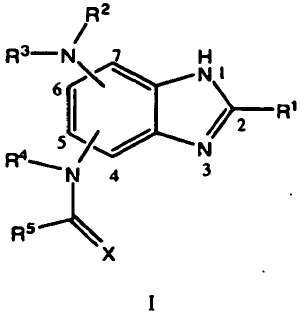 wherein:
R¹ represents NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CRR⁸OR⁶, CH₂NH₂, CR⁷R⁸NH₂, CR⁷R⁸NR⁹R¹⁰, alkyl, cycloalkyl, aryl, or halo;
R² and R⁴ independently represent H, alkyl, cycloalkyl, or aryl;
R⁵ represents H, R⁶. OR⁶, SR⁶, NH₂, NHR⁶, or NR⁹R¹⁰;
R⁶, R⁷, R⁸, R⁹, and R¹⁰ independently represent alkyl, cycloalkyl, or aryl;
R² and R³; R⁴ and R⁵; and R⁹ and R¹⁰ independently, may be combined to represent a heterocyclic alkyl or heterocyclic aryl;
R⁷ and R⁸ may be combined to represent a cycloalkyl;
and wherein:
X represents O and
R³ represents alkyl, cycloalkyl, aryl, CO(cycloalkyl) or CO(cycloaryl);
or
X represents S, NH, or NR⁶; and
R³ represents alkyl, cycloalkyl, aryl, or COR⁶;
or
X represents O;
R³ represents COCH₃; and
R¹, R², R⁴, R⁵ and R⁶ are as shown in the compounds of formula I below: 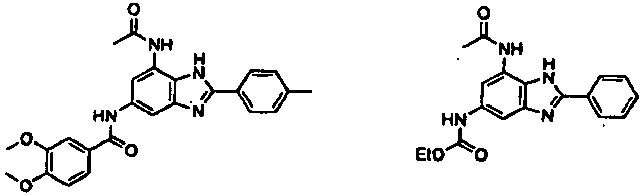 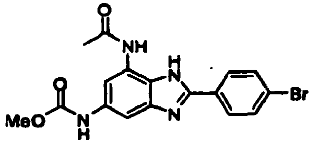 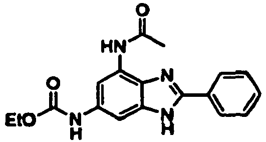 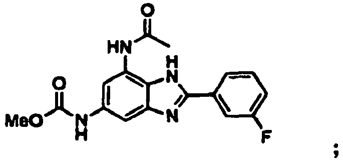
and wherein:
alkyl groups are branched or unbranched, saturated or unsaturated, and have 1-18 carbon atoms in their longest chain;
cycloalkyl groups are carbocyclic or heterocyclic, fused or unfused, non-aromatic ring systems having a total of 5-16 ring members including substituent rings;
aryl groups are carbocyclic or heterocyclic;
carbocyclic aryl groups are fused or unfused ring systems having a total of 6-16 ring members including substituent rings;
heterocyclic aryl groups are fused or unfused ring systems having a total of 5-16 ring members including substituent rings;
halo substituents are fluoro, chloro, or bromo;
each alkyl, cycloalkyl, and aryl, independently, may be unsubstituted or substituted with one or more substituent at any position;
alkyl substituents are halo, hydroxyl, OR⁶, SR⁶, NH₂, NHR⁶, NR⁹R¹⁰, cycloalkyl, or aryl;
cycloalkyl substituents are halo, hydroxyl, OR⁶, SR⁶, NH₂, NHR⁶, NR⁹R¹⁰, alkyl, cycloalkyl, or aryl;
aryl substituents are halo, hydroxyl, OR⁶, SR⁶, NH₂, NHR⁶, NR⁹R¹⁰, alkyl, cycloalkyl, aryl, nitro, or carboxyl; and
heterocyclic alkyl and heterocyclic aryl have at least one heteroatom selected from oxygen, nitrogen and sulfur, and
pharmaceutically acceptable salts thereof.

The invention also relates to the above-mentioned compounds of formula I or a pharmaceutically acceptable salt thereof for use in treating a patient infected with *Mycobaterium tuberculosis* or *Francisella tulerensis.*

### DETAILED DESCRIPTION

The invention relates to novel benzimidazole derivatives. These benzimidazole derivatives can be used to treat a patient infected by *Mycobacterium tuberculosis* or *Francisella tulerensis.*

The molecules in one aspect of the invention have formula I: 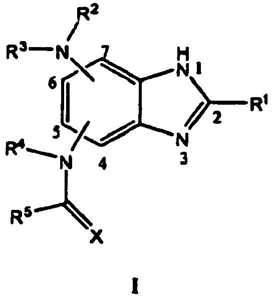 wherein:
R¹ represents NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, alkyl, cycloalkyl, aryl, or halo.
R² and R⁴ independently represent H, alkyl, cycloalkyl, or aryl. For example, R² may represent ethyl and R⁴ may represent H.
R³ represents alkyl, cycloalkyl, or aryl. For example, R³ may represent tetrahydrofuranyl or ethyl.

In a preferred embodiment, when R² represents H, R³ is not methyl.

R⁵ represents H, R⁶, OR⁶, SR⁶, NH₂, NHR⁶, or NR⁹R¹⁰, R⁶, R⁷, R⁸, R⁹, and R¹⁰ independently represent alkyl, cycloalkyl, aryl, or halo. Preferably, R⁶, R⁷, R⁸, R⁹, and R¹⁰ independently represent alkyl, cycloalkyl, or aryl. More preferably, R⁶, R⁷, R⁸, R⁹, and R¹⁰ independently represent alkyl or aryl.

R² and R³; R⁴ and R³; and R⁹ and R¹⁰, independently, may be combined to represent a heterocyclic, alkyl or heterocyclic aryl ring. For example, R² and R³ can be combined to represent a heterocyclic alkyl ring, resulting in the following structure: 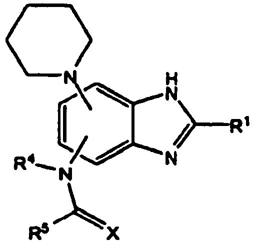 Similarly, R⁴ and R⁵ can be combined to represent a hetetocyclic alkyl ring, resulting in the following structure:

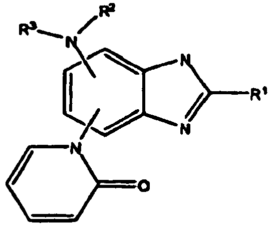

R⁷ and R⁸ may be combined to represent a cycloalkyl.

Alkyl groups are branched or unbranched, saturated or unsaturated, and have 1-18 carbon atoms in their longest chain. Some examples of suitable straight-chained, saturated alkyl groups include methyl, ethyl, *n*-propyl, n-butyl, n-pentyl, n-hexyl groups and dodecyl and hexadecyl. Preferred straight chain, saturated alkyl groups include methyl and ethyl.

Some examples of suitable branched, saturated alkyl groups include isopropyl, iso-butyl, sec-butyl, t-butyl, 1-methylbutyl, 2-methylbutyl, 3-methylbutyl (isopentyl), 1,1-dimethylpropyl, 1,2-dimethylpropyl, 2,2-dimethylpropyl (neopentyl), 1-methylpentyl, 2-methylpentyl, 3-methylpentyl, 4-methylpentyl groups, and 2-methyl,5-ethyldecyl. Preferred branched, saturated alkyl groups include isopropyl and t-butyl.

Some examples of unsaturated alkyl groups include ethenyl, ethynyl, propenyl, propargyl, isopropenyl, crotyl, 1-hexenyl, and 1-octenyl.

Cycloalkyl groups are carbocyclic or heterocyclic, fused or unfused, non-aromatic ring systems having a total of 5-16 ring members including substituent rings. Ring systems are monocyclic, bicyclic, tricyclic, or tetracyclic and can be bridged or non-bridged.

Some examples of carbocyclic alkyl groups include cyclobutanyl, cyclopentanyl, cyclohexanyl, and cycloheptanyl. Examples of fused carbocyclic alkyl groups include indenyl, isoindenyl. Bridged groups include bicyclo [2.2.1] heptane, bicyclco [5.2.0] nonane, and bicyclo [5.2.0] nonane.

Some examples of heterocyclic alkyl groups include pyrrolidinyl, piperidinyl, piperazinyl, tetrahydrofuranyl, morpholino, and oxazolidinyl. Examples of fused heterocyclic alkyl groups include benzomorpholino, benzopyrrolidinyl, indolinyl, and benzopiperidinyl.

Aryl groups can be either carbocyclic or heterocyclic.

Carbocyclic aryl groups are fused or unfused ring systems having a total of 6-16 ring members including substituent rings. A preferred unfused carbocyclic aryl group is phenyl.

Some examples of fused carbocyclic aryl groups include naphthyl, phenanthryl, anthracenyl, triphenylenyl, chrysenyl, and pyrenyl.

Heterocyclic aryl groups are fused or unfused ring systems having a total of 5-16 ring members including substituent rings.

Some examples of unfused heterocyclic aryl groups include thiophenyl, furyl, pyrrolyl, pyrazolyl, imidazolyl, oxazolyl, thiazolyl, pyridinyl, Pyridazinyl, pyrimidinyl, and pyrazinyl. Some examples of fused heterocyclic aryl groups include purinyl, 1,4-diazanaphthalenyl, indolyl, benzimidazolyl, 4,5-diazaphenanthrenyl, benzoxazolyl, isoindolyl, quinolinyl, isoquinolinyl, and benzofuranyl.

Halo substituents are fluoro, chloro, or bromo.

Each alkyl, cycloalkyl, and aryl, independently, may be unsubstituted or substituted with one or more substituent at any position. Alkyl substituents are halo, hydroxyl, OR⁶, SR⁶, NH₂, NHR⁶, NR⁹R¹⁰, cycloalkyl, or aryl. Cycloalkyl substituents are halo, hydroxyl, OR⁶, SR⁶, NH₂, NHR⁶, NR⁹R¹⁰, alkyl, cycloalkyl, or aryl. Aryl substituents are halo, hydroxyl, OR⁶, SR⁶, NH₂, NHR⁶, NR⁹R¹⁰, alkyl, cycloalkyl, aryl, nitro, or carboxyl.

Heterocyclic alky and heterocyclic aryl have at least one heteroatom selected from oxygen, nitrogen, and sulfur.

X represents O, S, NH, or NR⁶. R⁶ is described above.

The molecules in a further aspect of the invention have formula **I:** 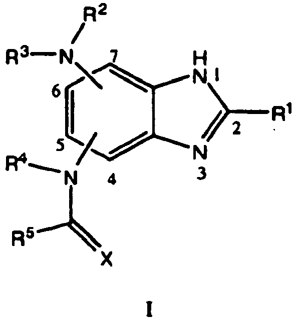 wherein:
R¹ represents NH₂, NHR⁶, NR⁹R¹⁰, NR⁶CONR⁹R¹⁰, NR⁶CSNR⁹R¹⁰, OH, OR⁶, SH, SR⁶, CHO, COOR⁶. COR⁶. CH₂OH, CR⁷R⁸OH, CH₂OR⁶, CR⁷R⁸OR⁶, CH₂NH₂, CR⁷R⁸NH₂, CR⁷R⁸NR⁹R¹⁰, alkyl, cycloalkyl, aryl, or halo;
R² and R⁴ independently represent H, alkyl, cycloalkyl, or aryl;
R⁵ represents H, R⁶, OR⁶, SR⁶, NH₂, NHR⁶, or NR⁹R¹⁰;
R⁶, R⁷, R⁸, R⁹, and R¹⁰ independently represent alkyl, cycloalkyl, or aryl;
R² and R³; R⁴ and R⁵; and R⁹ and R¹⁰ independently, may be combined to represent a heterocyclic alkyl or heterocyclic aryl;
R⁷ and R⁸ may be combined to represent a cycloalkyl;
and wherein:
X represents O; and
R³ represents alkyl, cycloalkyl, aryl, CO(cycloalkyl) or CO(cycloaryl);
or
X represents S, NH, or NR⁶; and
R³ represents alkyl, cycloalkyl, aryl, or COR⁶;
or
X represents O;
R³ represents COCH₃; and
R¹, R², R⁴, R⁵ and R⁶ are as shown in the compounds of formula I below:

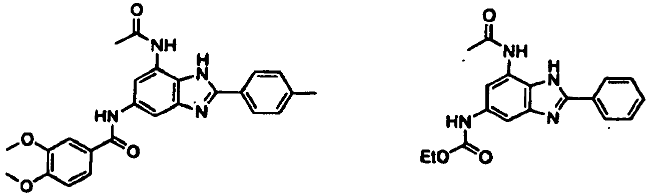

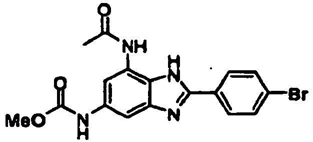

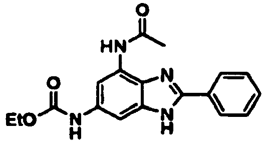

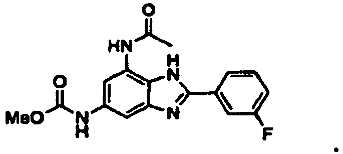

The nature of the alkyl groups, cycloalkyl groups and aryl groups, as well as their respective possible substituents, including halo substituents, are as described herein above in relation to formula I of the first aspect of the invention.

In the present invention, various parameters are defined (e.g. R¹, R², R³, R⁴, X). Within each parameter, more than one element (e.g. chemical moieties) are listed. It is to be understood that, unless specified otherwise, the instant invention contemplates embodiments in which each element listed under one parameter may be combined with each and every element listed under any other parameter. For example, X is identified above as representing O S, NH, or NR⁶. R⁵ is identified above as being H, R⁶, OR⁶, SR⁶, NH₂, NHR⁶, or NR⁹R¹⁰. Each element of X (O, S, NH or NR⁶) can be combined with each and every element of R⁵ (H, R⁶, OR⁶, SR⁶, NH₂, NHR⁶, or NR⁹R¹⁰). For example, in one embodiment, X may be O and R⁵ may be H. Alternatively, X may be NH and R⁵ may be NR⁹R¹⁰, etc. Similarly, a third parameter is R⁴, in which the elements are defined as H, alkyl, cycloalkyl, or aryl. Each of the above embodiments may be combined with each and every element of R⁴. For example, in the embodiment wherein X is O and R⁵ is H, R⁴ may be H (or any other chemical moiety within the element of R⁴)_{.}

The compounds of this invention are limited to those that are chemically feasible and stable. Therefore, a combination of substituents or variables in the compounds described above is permissible only if such a combination results in a stable or chemically feasible compound. A stable compound or chemically feasible compound is one in which the chemical structure is not substantially altered when kept at a temperature of 40°C or less, in the absence of moisture or other chemically reactive conditions, for at least a week.

### Pharmaceutically acceptable salts

The present invention also relates to pharmaceutically acceptable salts of the benzimidazole derivatives. The pharmaceutically acceptable salts include the conventional non-toxic salts of the benzimidazole derivatives as formed, e.g., from non-toxic inorganic or organic acids. For example, such conventional non-toxic salts include those derived from inorganic acids such as hydrochloric, hydrobromic, sulfuric, sulfamic, phosphoric, nitric and the like: and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like.

The pharmaceutically acceptable salts of the benzimidazole derivatives of this invention can be synthesized from the compounds of this invention which contain a basic moiety by conventional chemical methods. Generally, the salts are prepared either by ion exchange chromatography or by reacting the free base with stoichiometric amounts or with an excess of the desired salt-forming inorganic or organic acid in a suitable solvent or various combinations of solvents.

### Synthesis of the benzimidazole derivatives

The benzimidazoles of the present invention can be synthesized by methods known in the art. The following scheme represents one approach to the synthesis of the compounds of the invention.

Scheme I shows an example of a synthesis that yields individual compounds of the invention or a library of compounds of the invention. For example, the compounds of the invention may be made using polymer-assisted solution-phase (PASP) synthesis. PASP is a parallel synthesis method for creation of a trisubstituted benzimidazoles (BAZ-1) library using 2,4-dinitro-5-fluoroaniline (1) as the starting material.

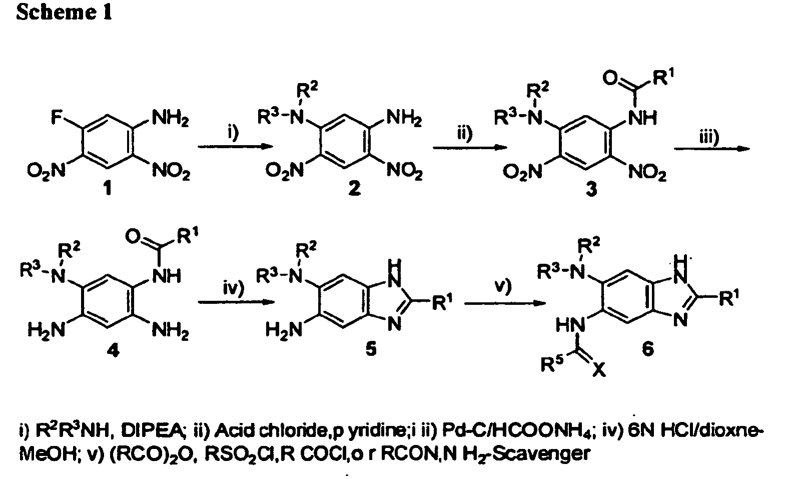

The first step involves the nucleophilic substitution of compound 1 with a secondary amine in the presence of N,N-diisopropylethylamine. The reaction produces compound 2 in high yields and purity at room temperature.

Then the acylation of the free amino group of compound 2 with an acyl or aroyl chloride takes place. This reaction occurs under reflux conditions using pyridine as the solvent.

Subsequently, reduction of the aromatic m-dinitro groups of compound 3 using HCOO⁻NH₄⁺ and Pd-C generates diamine compound 4. The benzimidazole ring is formed through acid-catalyzed dehydration.

The free aromatic amino group of compound 5 is modified in different ways. To introduce diversity at the -C(X)-R⁵ position, anhydride, acyl chloride, sulfonyl chloride, and isocyanate are used as modifying agents. The modification of the aromatic amine moiety takes place smoothly in dry dichloromethane and all excess acylating reagents are scavenged by commercially available aminomethylated polystyrene resin (from nova-biochem) to give the desired product 6 in 80-95% yield.

### Uses of the benzimidazole derivatives

The invention also relates to compounds of formula (I) or a pharmaceutically acceptable salt thereof for use in treating a patient infected with *Mycobacterium tuberculosis* or *Francisella tulerensis.*

The compounds of the invention may be employed alone, or in combination with other anti-bacterial agents. Other anti-bacterial agents include isoniazid, rifampin, pyrazinamide, rifabutin, streptomycin and ciprofloxacin. The combination of these anti-bacterial agents and the compounds of the invention will provide new agents for the treatment of tuberculosis, including MDR-TB and XDR-TB, and tularemia.

An effective amount of a compound of formula (I) or a pharmaceutically acceptable salt thereof as used herein is any amount effective to treat a patient infected by *Mtb* or *F*. *tulerensis.* Modes of administration and doses can be determined by those having skill in the art. An effective amount of the compound will vary with the group of patients (age, sex, weight, etc.), the nature and severity of the condition to be treated, the particular compound administered, and its route of administration. Amounts suitable for administration to humans are routinely determined by physicians and clinicians during clinical trials.

The minimum dose of the compound is the lowest dose at which efficacy is observed. For example, the minimum dose of the compound may be about 0.1 mg/kg/day, about 1 mg/kg/day, or about 3 mg/kg/day.

The maximum dose of the compound is the highest dose at which efficacy is observed in a patient, and side effects are tolerable. For example, the maximum dose of the compound may be about 10 mg/kg/day, about 9 mg/kg/day, or about 8 mg/kg/day.

A benzimidazole derivative of the present invention may be administered by any method known in the art. Some examples of suitable modes of administration include oral and systemic administration. Systemic administration can be enteral or parenteral. Liquid or solid (e.g., tablets, gelatin capsules) formulations can be employed.

Parenteral administration of the benzimidazole derivative include, for example intravenous, intramuscular, and subcutaneous injections. For instance, a chemical compound may be administered to a patient by sustained release, as is known in the art. Sustained release administration is a method of drug delivery to achieve a certain level of the drug over a particular period of time.

Other routes of administration include oral, topical, intrabronchial, or intranasal administration. For oral administration, liquid or solid formulations may be used. Some examples of formulations suitable for oral administration include tablets, gelatin capsules, pills, troches, elixirs, suspensions, syrups, and wafers. Intrabronchial administration can include an inhaler spray. For intranasal administration, administration of a chemical compound can be accomplished by a nebulizer or liquid mist.

The chemical compound can be formulated in a suitable pharmaceutical carrier. In this specification, a pharmaceutical carrier is considered to be synonymous with a vehicle or an excipient as is understood by practitioners in the art. Examples of carriers include starch, milk, sugar, certain types of clay, gelatin, stearic acid or salts thereof, magnesium or calcium stearate, talc, vegetable fats or oils, gums and glycols.

The chemical compound can be formulated into a composition containing one or more of the following: a stabilizer, a surfactant preferably a nonionic surfactant, and optionally a salt and/or a buffering agent.

The stabilizer may, for example, be an amino acid, such as for instance, glycine; or an oligosaccharide, such as for example, sucrose, tetralose, lactose or a dextran. Alternatively, the stabilizer may be a sugar alcohol, such as for instance, mannitol; or a combination thereof. Preferably the stabilizer or combination of stabilizers constitutes from about 0.1% to about 10% weight for weight of the chemical compound.

The surfactant is preferably a nonionic surfactant, such as a polysorbate. Some examples of suitable surfactants include Tween 20, Tween 80; a polyethylene glycol or a polyoxyethylene polyoxypropylene glycol, such as Pluronic F-68 at from about 0.001% (w/v) to about 10% (w/v).

The salt or buffering agent may be any salt or buffering agent, such as for example sodium chloride, or sodium/potassium phosphate, respectively. Preferably, the buffering agent maintains the pH of the chemical compound formulation in the range of about 5.5 to about 7.5. The salt and/or buffering agent is also useful to maintain the osmolality at a level suitable for administration to a patient. Preferably the salt or buffering agent is present at a roughly isotonic concentration of about 150 mM to about 300 mM.

The chemical compound can be formulated into a composition which may additionally contain one or more conventional additives. Some examples of such additives include a solubilizer such as, for example, glycerol; an antioxidant such as for example, benzalkonium chloride (a mixture of quaternary ammonium compounds, known as "quart"), benzyl alcohol, chloretone or chlorobutanol; anaesthetic agent such as, for example a morphine derivative; or an isotonic agent etc. As a further precaution against oxidation or other spoilage, the composition may be stored under nitrogen gas in vials sealed with impermeable stoppers.

### EXAMPLES

Examples have been set forth below for the purposes of illustration and to describe the best mode of the invention at the present time. The scope of the invention is not to be in any way limited by the examples set forth herein.

### EXAMPLE 1

### Synthesis of 6-diethylamino-5-(4-methoxybenzoyl)amino-2-(2-methoxyphenyl)-1H-benzo[d]imidazole, a key intermediate for the synthesis of a library of compounds (the process includes three steps):

### (a) Synthesis of 1-amino-3-diethylamino-4,6-dinitrobenzene:

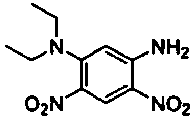

To a solution of 2,4-dinitro-5-fluoroaniline (1.6g, 8.0 mmol) in 20 mL of THF, a mixture of DIPEA (1.1 g, 8.8 mmol) and diethylamine (644mg, 8.8 mmol) in 5 mL of THF was added slowly. The reaction mixture was stirred at room temperature for 1 h. Water (100 mL) was added to give the desired product as a yellow precipitate. The product was collected by filtration and washed with water (200mL). The filtrate was concentrated to dryness in vacuo to give the desired product (1.8 g, 90 % yield) as a bright yellow solid: ¹H-NMR (300MHz, CDCl₃) δ 1.96 (t, 6H, J = 7.2 Hz), 3.24 (q, 4H, J = 7.2 Hz), 6.08 (s, 1H), 8.75 (s, 1H); ¹³C NMR (75MHz, CDCl₃) δ12.1, 45.7, 102.7, 123.3, 128.2, 131.6, 147.8, 149.4; ESI MS m/z 255.1 [M+H]⁺.

### (b) Synthesis of 5-(diethylamino)-2,4-dinitro-1-(2-methoxybenzoyl)aminobenzene:

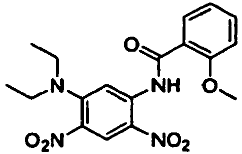

To a solution of 1-amino-3-diethylamino-4,6-dinitrobenzene (508mg, 2.0 mmol) in 5 mL of pyridine, 2-methoxybenzoyl chloride (680mg, 4.0 mmol) was added. After refluxing for 5 h, 50 mL of water was added to the reaction mixture, and the precipitate was collected by filtration and washed with 200 mL of water. Recrystallization from dichloromethane and methanol gave the desired product (622 mg, 80% yield) as a yellow solid: ¹H-NMR (300MHz, CDCl₃) δ 1.29 (t, 6H, J = 7.2 Hz), 3.39 (q, 4H, J = 7.2 Hz), 4.13 (s, 3H), 7.07 (d, 1H, J = 8.1), 7.11 (t, 1H, J = 9.8 Hz), 7.55 (t, 1H, J = 7.8 Hz), 8.23 (d, 1H, J = 8.1 Hz), 8.83 (s, 1H), 8.98 (s, 1H); ESI MS m/z 389.1 [M+H]⁺.

### (c) Synthesis of 5-amino-6-diethylamino-2-(2-methoxyphenyl)-1H-benzo[d]-imidazole:

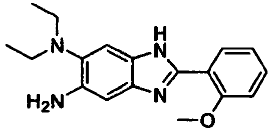

To the solution of 5-(diethylamino)-2,4-dinitro-1-(2-methoxybenzoyl)aminobenzene (388mg, 1.0 mmol) in 10 mL of 1,4-dioxane and 10 mL of methanol, was added ammonium formate (1.5g) and 10% Pd/C (200 mg) under nitrogen atmosphere. The reaction mixture was stirred for 30 min. The Pd/C and excess ammonium formate were filtered. Conc. HCl (10 mL) was added to the filtrate. After heating at 75 °C for 18 h, the reaction mixture was basified to pH 8 with saturated K₂CO₃ solution. The reaction mixture was diluted with 200 mL of ethyl acetate, washed with brine, and dried over anhydrous MgSO₄. The reaction mixture was filtered and concentrated in vacuo to afford the crude product (280mg, 90% yield). The crude product was then purified by column chromatography on slica gel using EtOAc as the eluant to afford the desired product (188 mg, 61% yield) as a brown solid: ¹H-NMR (300MHz, CDCl₃) δ 1.01 (t, 6H, J = 7.2 Hz), 3.01 (q, 4H, J = 7.2 Hz), 4.05 (s, 3H), 6.92 (s, 1H), 7.03 (d, 1H, J = 8.1 Hz), 7.11 (t, 1H, J = 8.1 Hz), 7.36 (t, 1 H, J = 6.9), 7.41 (s, 1H), 8.52 (d, 1H, J = 7.8 Hz); ESI MS m/z 311.2 [M+H]⁺.

### EXAMPLES 2-7

The following key intermediates were prepared and characterized in the same manner as Example 1.

### 5-Amino-6-diethylamino-2-(cyclohexyl)-1H-benzo[d]-imidazole:

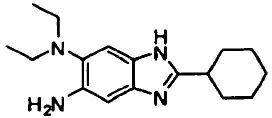

Brown solid; ¹H-NMR (300MHz, CDCl₃) δ 0.95 (t, 6H, J = 7.2 Hz), 1.2-2.2 (m, 10H), 2.82 (m, 1H), 2.92 (q, 4H, J = 7.2 Hz), 6.90 (s, 1H), 7.33 (s, 1H); ESI MS m/z 287.1 [M+H]⁺.

### 5-Amino-6-diethylamino-2-(4-fluorophenyl)-1H-benzo[d]-imidazole:

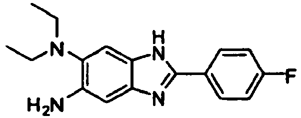

Brown solid; ¹H-NMR (300MHz, CDCl₃) δ 0.94 (t, 6H, J = 7.2 Hz), 2.91 (q, 4H, J = 7.2 Hz), 6.80 (s, I H), 7.02 (t, 2H, J = 8.7 Hz), 7.27 (s, 1H), 7.98 (ddd, 2H, J = 1.8, 5.4, 8.7 Hz); ESI MS m/z 299.1 [M+H]⁺.

### 5-Amino-6-diethylamino-2-(phenyl)-1H-benzo[d]-imidazole:

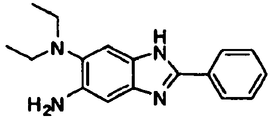

Brown solid; ¹H-NMR (300MHz, CDCl₃) δ 0.92 (t, 6H, J = 7.2 Hz), 2.92 (q, 4H, J = 7.2 Hz), 6.85 (s, 1H), 7.32 (s, 1H), 7.41 (m, 3H), 8.01 (dd, 2H, J = 1.8, 8.4 Hz); ESI MS m/z 281.1 [M+H]⁺.

### 5-Amino-6-diethylamino-2-(4-methylphenyl)-1H-benzo[d]-imidazole:

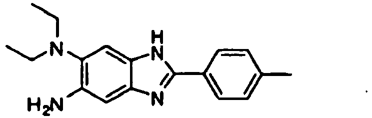

Brown solid; ¹H-NMR (300MHz, CDCl₃) δ 0.91 (t, 6H, J = 7.2 Hz), 2.90 (q, 4H, J = 7.2 Hz), 2.33 (s, 3H), 6.79 (s, 1H), 7.13 (d, 2H, J = 7.8 Hz), 7.32 (s, 1H), 7.99 (d, 2H, J = 7.8 Hz); ESI MS m/z 295.1 [M+H]⁺.

### 5-Amino-6-diethylamino-2-(4-methoxyphenyl)-1H-benzo[d]-imidazole:

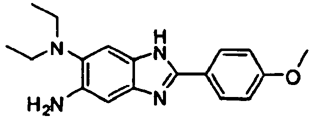

Brown solid; ¹H-NMR (300MHz, CDCl₃) δ 0.93 (t, 6H, J = 7.2 Hz), 2.92 (q, 4H, J = 7.2 Hz), 4.03 (s, 3H), 6.73 (s, 1H), 6.75 (d, 2H, J = 8.4 Hz), 7.10 (d, 2H, J = 8.4 Hz) 7.16 (s, 1H); ESI MS m/z 311.1 [M+H]⁺.

### 5-Amino-6-diethylamino-2-(1-naphthyl)-1H-benzo[d]-imidazole:

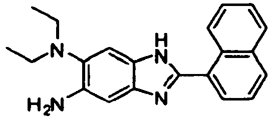

Brown solid; ¹H-NMR (300MHz, CDCl₃) δ 0.91 (t, 6H, J = 7.2 Hz), 2.92 (q, 4H, J = 7.2 Hz), 6.65 (s, 1H), 7.10 (s, 1H), 7.35 (t, 1H, J = 7.5 Hz), 7.44 (t, 2H, J = 4.2 Hz), 7.67 (d, 1H, J = 6.3), 7.83 (m, 2H), 8.64 (m, 1H); ESI MS m/z 349.2 [M+H]⁺.

### EXAMPLE 8

### Synthesis of 6-diethylamino-5-(4-methoxybenzoyl)amino-2-(2-methoxyphenyl)-1H-benzo[d]imidazole:

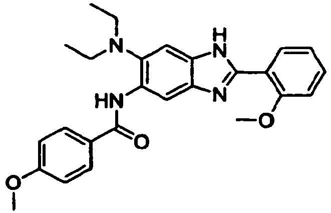

To a solution of 5-amino-6-diethylamino-2-(2-methoxyphenyl)-1H-benzo[d]-imidazol (200 mg, 0.64 mmol) in dichloromethane (5 mL), 2-methoxybenzoyl chloride (112 mg, 0.64 mmol) was added and stirred at room temperature for 3 h. The reaction mixture was concentrated in vacuo and then purified by column chromatography on slica gel using hexane/EtOAc (4/1) as the eluant to afford the desired product (210 mg, 78%) as a white powder: ¹H-NMR (300MHz, CDCl₃) δ 1.00 (t, 6H, J = 7.2 Hz), 3.05 (q, 4H, J = 7.2 Hz), 3.86 (s, 3H), 4.04 (s, 3H), 6.85 (d, 2H, J = 9 Hz), 7.03 (d, 1H, J = 8.7 Hz), 7.13 (t, 1H, J = 7.2 Hz), 7.39 (t, 1H, J = 7.2 Hz), 7.66 (s, 1H), 7.91 (d, 2H, J = 9Hz), 8.88 (s, 1H); ¹³C NMR (75MHz, CDCl₃) δ12.9, 50.4, 55.3, 55.7, 111.4, 113.8, 117.9, 121.5, 127.8, 128.6, 129.6, 130.8, 132.8, 135.8, 149.9, 156.6, 162.1, 164.1; ESI MS m/z 445.4 [M+H]⁺ .

### EXAMPLE 9

### Synthesis of 6-diethylamino-5-(4-chlorobenzoyl)amino-2-(2-methoxyphenyl)-1H-benzo[d]imidazole:

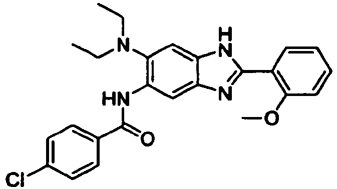

5-Amino-6-diethylamino-2-(2-methoxyphenyl)-1H-benzo[d]-imidazole (187 mg, 0.6 mmol) was reacted with 4-chlorobenzoyl chloride (105 mg, 0.6 mmol) in the same manner as that described above to give the desired product (216 mg, 80% yield) as pale yellow powder: ¹H-NMR (300MHz, CDCl₃) δ 0.99 (t, 6H, J = 7.2 Hz), 3.05 (q, 4H, J = 7.2 Hz), 4.07 (s, 3H), 7.05 (d, 1H, J = 8.4 Hz), 7.11 (t, 1H, J = 7.8 Hz), 7.41 (t, 1H, J = 7.8 Hz), 7.47 (d, 2H, J = 8.4 Hz), 7.67 (s, 1H), 7.88 (d, 2H, J = 8.4 Hz), 8.55 (d, 1H, J = 6.9 Hz), 8.86 (s, 1H); ¹³C NMR (75MHz, CDCl₃) δ13.1, 50.6, 55.9, 111.5, 117.7, 121.7, 128.3, 129.0, 129.8, 131.1, 132.5, 133.9, 136.0, 137.7, 150.2, 156.7, 163.4; ESI MS m/z 449.2 [M+H]⁺.

### EXAMPLE 10

### Synthesis of 6-diethylamino-5-(benzoyl)amino-2-(cyclohexy)-1H-benzo[d]-imidazole:

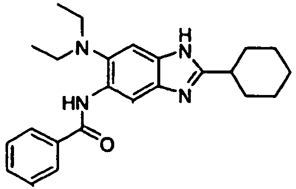

5-Amino-6-diethylamino-2-(cyclohexyl)-1H-benzo[d]-imidazole (27 mg, 0.1 mmol) was reacted with benzoyl chloride (11 mg, 0.1 mmol) in the same manner as that described above to give the desired product (27 mg, 74 % yield) as white powder: ¹H-NMR (300MHz, CDCl₃) δ 0.98 (t, 6H, J = 7.2 Hz), 1.16 (m, 3H), 1.57-1.70 (m, 5H), 1.98 (m, 2H), 2.87 (m, 1H), 3.03 (q, 4H, J = 7.2 Hz), 7.57 (m, 3H), 7.57 (s, 1H), 8.00 (dd, 2H, J = 1.8, 8.4 Hz), 8.96 (s, 1H); ESI MS m/z 391.0 [M+H]⁺.

### EXAMPLE 11

### Synthesis of 6-diethylamino-5-(4-methoxybenzoyl)amino-2-(cyclohexyl)-1H-benzo[d]imidazole:

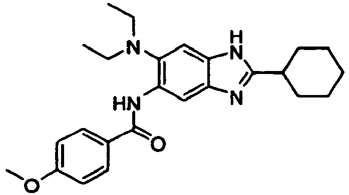

5-Amino-6-diethylamino-2-(cyclohexyl)-1H-benzo[d]-imidazole (28 mg, 0.1 mmol) was reacted with 4-methoxybenzoyl chloride (17 mg, 0.1 mmol) in the same manner as that described above to give the desired product (33 mg, 79% yield) as white powder: ¹H-NMR (300MHz, CDCl₃) δ 0.97 (t, 6H, J = 7.2 Hz), 1.16 (m, 3H), 1.57-1.70 (m, 5H), 1.98 (m, 2H), 2.87 (m, 1H), 3.01 (q, 4H, J = 7.2 Hz), 7.05 (d, 2H, J = 8.7 Hz), 7.57 (s, 1H), 7.96 (d, 2H, J = 8.7 Hz), 8.94 (s, 1H); ESI MS m/z 421.0 [M+H]⁺.

### EXAMPLE 12

### Synthesis of 7-acetylamino-5-(methoxycarbonyl)amino-2-(4-bromophenyl)-1H-benzo[d]-imidazole (the process includes three steps):

### (a) Synthesis of 4-amino-3,5-dinitro-1-(methoxycarbonyl)aminobenzene:

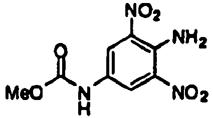

A suspension of 4-amino-3,5-dinitrobenzamide (543 mg, 2.4 mmol) in 4M HCl (20 mL) was refluxed overnight. The reaction mixture was cooled and the precipitated solid was filtered to give 4-amino-3,5-dinitrobenzoic acid as yellow solid: ¹H-NMR (300 MHz, DMSO-d₆) δ 8.80(s, 2H). 4-Amino-3,5-dinitrobenzoic acid, thus obtained, was dissolved in SOCl₂ (4 mL) and refluxed overnight. The reaction mixture was cooled down to room temperature and concentrated under reduced pressure to remove excess SOCl₂. The crude product was immediately dissolved in acetone (2.4 mL) in an ice-bath. To this solution was added dropwise NaN₃ (0.29 g, 3.84 mmol) in ice-water (0.88 mL). The mixture was stirred for 20 min at 0 °C until a solid precipitated out. After dilution with ice-water (12 mL), the reaction mixture was extracted with CH₂Cl₂ (6 mL x 2), dried over MgSO₄ at 0 °C for 1 h, and filtered. The filtrate was concentrated on a rotary evaporator (below room temperature), and the residue dissolved in toluene (15 mL). After refluxing for 2 h, the reaction mixture was cooled down to room temperature, and MeOH (10 mL) was added. After stirring overnight at room temperature, the reaction mixture was concentrated in vacuo and purified by flash chromatography on silica gel (hexane/EtOAc = 1/1) to afford 4-amino-3,5-dinitro-1-(methoxycarbonyl)aminobenzene as bright red solid (292 mg, 45% yield): ¹H-NMR (300 MHz, CDCl₃) δ 3.73 (s, 3H), 6.60(s, 1 H), 8.30(s, 2 H), 8.64(s, 2 H); ESI MS m/z 256.9 [M+H]⁺.

### (b) Synthesis of 7-amino-5-(methoxycarbonyl)amino-2-(4-bromophenyl)-1H-benzo[d]imidazole:

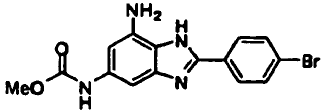

To a suspension of 4-amino-3,5-dinitro-1-(methoxycarbonyl)aminobenzene (311 mg, 1.2 mmol) in ethanol (24 mL), was added ammonium formate (1.8 g) and 10% Pd/C (120 mg) under nitrogen. The mixture was stirred at room temperature overnight. The Pd/C and excess ammonium formate were filtered off. The filtrate was treated with the sodium bisulfite adduct of 4-bromobenzaldehyde (715 mg, 0.84 mmol) at 0 °C. After the solution was stirred for 12-16 h at room temperature under nitrogen, a trace of insoluble material was removed by filtration and the filtrate was concentrated on a rotary evaporator until approximately 60-70% of the solvent was removed. To the residue an equal volume of ethyl acetate was added, and the mixture was transferred to a separatory funnel. The organic layer was separated, and the water layer was extracted with ethyl acetate. The combined organic layers were washed with brine, dried over anhydrous sodium sulfate, and concentrated in vacuo to give the desired product (610 mg, 48% yield) as brown powder: ¹H-NMR (300MHz, CD₃OD) δ 3.73 (s, 3H), 6.53 (s, 1H), 7.16 (s, 1H), 7.64 (dd, 2H, J = 6.6, 1.8 Hz), 7.88 (dd, 2H, J = 6.6, 1.8 Hz); ESI MS m/z 361.0 [M+H]⁺.

### (c) Synthesis of 7-acetylamino-5-(methoxycarbonyl)amino-2-(4-bromophenyl)-1H-benzo[d]imidazole:

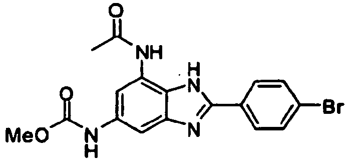

To a solution of 7-amino-5-(methoxycarbonyl)amino-2-(4-bromophenyl)-1H-benzo[d]-imidazole (60 mg, 0.17 mmol) in dichloromethane (5 mL) was added acetic anhydride (18 mg, 0.17 mmol) and the solution was stirred at room temperature for 3 h. The reaction mixture was concentrated in vacuo and purified by column chromatography on slica gel using hexane/EtOAc (4/1) as the eluant to afford the desired product (55 mg, 80%) as a pale yellow powder: ¹H-NMR (300MHz, CD₃OD) δ 2.24 (s, 3H), 3.74 (s, 3H), 7.65 (m, 3H), 7.67(bs, 1H), 7.90 (m, 2H) ; ESI MS m/z 403.0 [M+H]⁺.

### EXAMPLE 13

Procedure for the determination of the minimum inhibitory concentration (MIC): MIC values were determined using the microplate dilution method, previously reported [R. A. Slayden and C. E. Barry, III. "The role of KasA and KasB in the biosynthesis of meromycolic acids and isoniazid resistance in Mycobacterium tuberculosis", Tuberculosis (Edinb) 82:149-60 (2002)].

*Bacteria* were cultivated in liquid medium to an optical density of ~0.4 at 600 nm. The bacterial cultures were then prepared for testing by diluting 1:100 in liquid medium. A total of 50 µL of each culture was added to each well of a 96-well optical plate. Analogs were prepared at 60 µM in 100% DMSO. Compound stock solutions were diluted 1:2 in liquid medium and then distributed in the plate as 2-fold serial dilutions to achieve a concentration range of 200-0.2 mg/mL in a total final volume of 100 µL. The plates were incubated at 37°C and evaluated for the presence of bacterial growth or non-growth by optical density using an inverted plate reading method. The MIC₉₉ was determined to be the lowest concentration of compound that inhibited bacterial growth. Reported MIC values represent measurements performed independently in triplicate.

A list of active compounds is included in the Appendix section.

### PREPARATIVE EXAMPLES 14-16

The following key intermediates were prepared and characterized in the same manner as Example 1(a).

### 1-Amino-2,4-dinitro-5-morpholinobenzene:

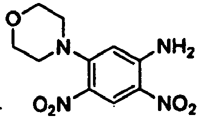

Yield 92 %; ¹HNMR (400 MHz, CDCl₃) δ 8.92 (s, 1 H) 6.12 (s, 1 H), 3.86 (t, 4 H, *J* = 6.2 Hz), 3.12 (t, 4 H, *J* = 6.2 Hz); ESI MS m/z 269.2 [M+H]⁺.

### 1-Amino-2,4-dinitro-5-piperidinobenzene:

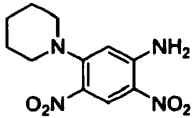

Yield 94 %; ¹HNMR (300 MHz, CDCl₃) δ 8.84 (s, 1 H) 6.43 (bs, 2 H), 3.09 (t, 4 H, *J* = 5 Hz), 1.71 (m, 4 H); ESI MS m/z 267.2 [M+H]⁺.

### 1-Amino-2,4-dinitro-5-(4-tert-butoxycarbonylpiperazino)benzene:

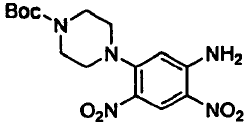

Yield 94 % ¹HNMR (400 MHz, CDCl₃) δ 8.91 (s, 1 H) 6.16 (s, 1 H), 3.61 (t, 4 H, *J* = 5 Hz), 3.09 (t, 4 H, *J* = 4.8 Hz), 1.47 (s, 9 H); ESI MS m/z 368.3 [M+H]⁺.

### PREPARATIVE EXAMPLES 17-29

The following key intermediates were prepared and characterized in the same manner as Example I (b).

### 1-Cyclohexanecarbonylamino-5-diethylamino-2,4-dinitrobenzene:

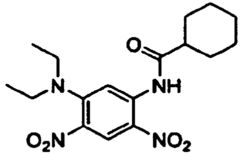

Yield 88 %; ¹HNMR (300 MHz, CDCl₃) δ 8.76 (s, 1 H), 8.65 (s,1 H), 3.36 (q, 4 H, *J* = 10.8 Hz), 2.38 - 1.30 (m, 11 H), 1.26 (t, 6 H, *J* = 7.2 Hz); ESI MS m/z 365.4 [M+H]⁺.

### 1-(4-Methylbenzoyl)amino-5-diethylamino-2,4-dinitrobenzene:

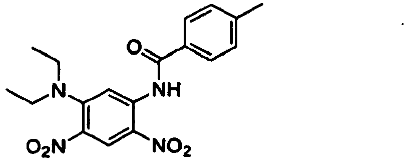

Yield 67 %; ¹HNMR (300 MHz, CDCl₃) δ 8.8 (d, 2 H, *J=* 5.8 Hz), 7.87 (d, 2 H, *J =* 4 Hz), 7.33 (d, 2 H, *J* = 4 Hz), 3.4 (q, 4 H, *J =* 10.6 Hz), 2.43 (s, 3 H), 1.29 (t, 6 H, *J =* 7.2 Hz); ESI MS m/z 373.3 [M+H]⁺.

### 1-(4-Methoxybenzoyl)amino-5-diethylamino-2,4-dinitrobenzene:

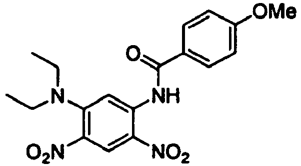

Yield 85 %; ¹HNMR (300 MHz, CDCl₃) δ 11.79 (s, 1 H), 8.82 (d, 2 H, *J* = 2.1 Hz), 7.96 (d, 2 H, *J =* 4.5 Hz), 7.03 (d, 2 H, *J =* 4.3 Hz), 3.39 (s, 3 H), 3.41 (q, 4 H, *J* = 10.6 Hz), 1.30 (t, 6 H, *J* = 6.9 Hz); ESI MS m/z 389.3 [M+H]⁺.

### 1-(4-tert-Butylbenzoyl)amino-5-diethylamino-2,4-dinitrobenzene:

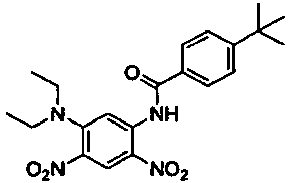

Yield 78 %; ¹HNMR (300 MHz, CDCl₃) δ 8.84 (s, 1 H), 8.82 (s, 1 H), 7.94 (d, 2 H, *J* = 4.3 Hz), 7.56 (d, 2 H, *J* = 4.2 Hz), 3.41 (q, 4 H, *J* = 10.6 Hz), 1.37 (s, 9 H), 1.30 (t, 6 H, *J =* 7.2 Hz); ESI MS m/z 415.4 [M+H]⁺.

### 1-(4-Fluorobenzoyl)amino-5-diethylamino-2,4-dinitrobenzene:

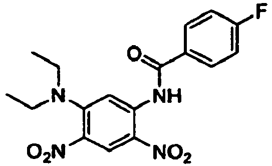

Yield 79 %; ¹HNMR (300 MHz, CDCl₃) δ 11.84 (s, 1 H), 8.81 (s, 1 H), 8.8 (s, 1 H), 8.02 (d, 2 H, *J* = 7.2 Hz), 7.22 (d, 2 H, *J* = 5.2 Hz), 3.41 (q, 4 H, *J* = 10.6 Hz), 1.30 (t, 6 H, *J* = 7.2 Hz); ESI MS m/z 377.3 [M+H]⁺.

### 1-Benzoylamino-5-diethylamino-2,4-dinitrobenzene:

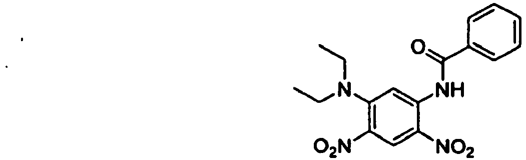

Yield 80 %; ¹HNMR (300 MHz, CDCl₃) δ 11.87 (s, 1 H), 8.84 (s, 1 H), 8.82 (s, 1 H), 8.0 (d, 2 H, *J* = 4.8 Hz), 7.63-7.55 (m, 3 H), 3.41 (q, 4 H, *J* = 10.6 Hz), 1.31 (t, 6 H, *J* = 7.2 Hz); ESI MS m/z 359.3 [M+H]⁺.

### 1-Cyclobexanecarbonylamino-5-morpholino-2,4-dinitrobenzene:

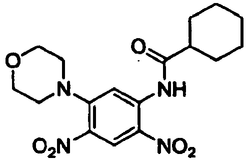

Yield 90 %; ¹HNMR (400 MHz, CDCl₃) δ 10.92 (s, 1 H), 8.87 (s, 1 H), 8.64 (s, 1 H), 3.83 (t, 4 H, *J=* 4.8 Hz), 3.28 (t, 4 H, *J=* 4.6 Hz), 2.38 (m, 1 H), 2.03-1.26 (m, 11 H); ESI MS m/z 379.3 [M+H]⁺.

### 1-(4-Methylbenzoyl-5-morpholino-2,4-dinitrobenzene:

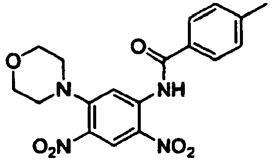

Yield 87 %; ¹HNMR (400 MHz, CDCl₃) δ 11.80 (s, 1 H), 8.95 (s, 1 H), 8.83 (s, 1 H), 7.88 (d, 2 H, *J* = 4.2 Hz), 7.36 (d, 2 H, *J* = 4 Hz), 3.88 (t, 4 H, *J* = 4.6 Hz), 3.34 (t, 4 H, *J* = 4.6 Hz), 2.46 (s, 3 H); ESI MS m/z 387.3 [M+H]⁺.

### 1-(4-tert-Butylbenzoyl)amino-5-morpholino-2,4-dinitrobenzene:

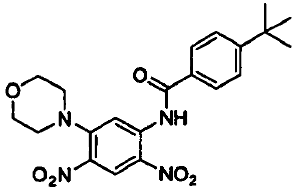

Yield 75 %; ¹HNMR (400 MHz, CDCl₃) δ 11.81 (s, 1 H), 8.95 (s, 1 H), 8.84 (s, 1 H), 7.92 (d, 2 H, *J* = 4.2 Hz), 7.57 (d, 2 H, *J* = 4.2 Hz), 3.88 (t, 4 H, *J* = 4.6 Hz), 3.34 (t, 4 H, *J* = 4.6 Hz), 1.37 (s, 9 H); ESI MS m/z 429.4 [M+H]⁺.

### 1-Cyclohexanecarbonylamino-2,4-dinitro-5-(4-tert-butoxycarbonylpiperazin-1-yl)benzene:

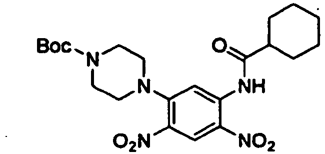

Yield 70 %; ¹HNMR (400 MHz, CDCl₃) δ 10.92 (s, 1 H), 8.90 (s, 1 H), 8.67 (s, 1 H), 3.62 (t, 4 H, *J =* 5.2 Hz), 3.28 (t, 4 H, *J =* 5.2 Hz), 2.38 (m, 1 H), 2.03-1.26 (m, 11 H); ESI MS m/z 478.5 [M+H]⁺.

### 1-Cyclohexanecarbonylamino-2,4-dinitro-5-(piperidin-1-yl)benzene:

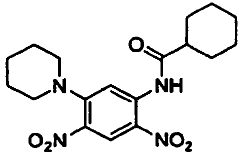

Yield 80 %; ¹HNMR (300 MHz, CDCl₃) δ 10.95 (s, 1 H), 8.85 (s, I H), 8.63 (s, 1 H), 3.27 (t, 4 H, *J* = 4.9 Hz), 2.38 (m, 1 H), 2.03-1.26 (m, 16 H); ESI MS m/z 377.4 [M+H]⁺.

### 1-(4-Methoxybenzoyl)amino-2,4-dinitro-5-(piperidin-1-yl)benzene:

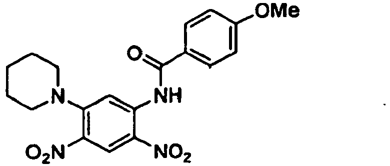

Yield 85 %; ¹HNMR (300 MHz, CDCl₃) δ 11.69 (s, 1 H), 8.90 (s, 1 H), 8.79 (s, 1 H), 7.95 (d, 2 H, *J =* 4.5 Hz), 7.03 (d, 2 H, *J* = 4.3 Hz), 3.90 (s, 3 H), 3.32 (t, 4 H, *J* = 4.95 Hz), 1.75 (m, 6 H); ESI MS m/z 401.3 [M+H]⁺.

### 1-Benzoylamino-2,4-dinitro-5-(piperidin-1-yl)benzene:

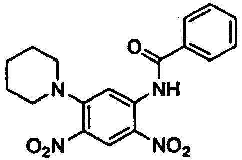

Yield 83 %; ¹HNMR (300 MHz, CDCl₃) δ 11.87 (s, 1 H), 8.91 (s, 1 H), 8.80 (s, 1 H), 7.98 (d, 2 H, *J* = 4.8 Hz), 7.66-7.55 (m, 3 H), 3.33 (t, 4 H, *J* = 4.9 Hz), 1.76 (m, 6 H); ESI MS m/z 371.3 [M+H]⁺.

### EXAMPLES 30-34

The following key intermediates were prepared and characterized in the same manner as Example 1(c).

### 5-Amino-2-cyclohexyl-6-diethylaminobenzo[d]imidazole:

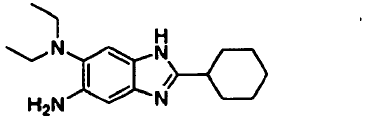

Yield 55 %; ¹HNMR (300 MHz, CDCl₃) δ 7.31 (s, 1 H), 6.9 (s, 1 H), 2.92 (m, 4 H, *J* = 10.8 Hz), 2.04 (m, 2 H), 1.68 (m, 5 H), 1.26 (m, 4 H), 0.95 (t, 6 H, *J* = 6.9 Hz); ESI MS m/z 287.4 [M+H]⁺.

### 5-Amino-6-diethylamino-2-(4-methylphenyl)-1H-benzo[d]imidazole:

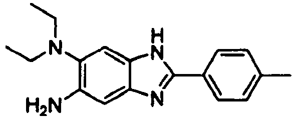

Yield 46 %; ¹HNMR (400 MHz, CDCl₃) δ 7.95 (d, *J* = 4.2 Hz, 2 H), 7.21 (s, 1 H), 7.12 (d, 2 H, *J* = 4 Hz), 6.77 (s, 1 H), 2.84 (q, 4 H, *J* = 10.6 Hz), 2.3 (s, 3 H), 0.90 (t, 6 H, *J* = 7 Hz); ESI MS m/z 295.3 [M+H]⁺.

### 5-Amino-6-diethylamino-2-(2-methoxyphenyl)-1H-benzo[d]imidazole:

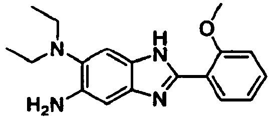

Yield 52 %; ¹HNMR (400 MHz, CDCl₃) δ 8.48 (dd, 1 H), 7.37-7.29 (m, 2 H), 7.48 (t, 1 H, *J* = 8 Hz), 6.97 (d, 1 H, *J* = 4.2 Hz), 6.88 (s, 1 H), 3.96 (s, 3 H), 2.96 (q, 4 H, *J* = 10.6 Hz), 0.97 (t, 6 H, *J* = 7 Hz); ESI MS m/z 311.3 [M+H]⁺.

### 5-Amino-6-diethylamino-2-(4-tert-butylphenyl)-1H-benzo[d]imidazole:

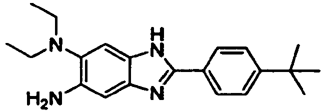

Yield 50 %; ¹HNMR (400 MHz, CDCl₃) δ 8.01 (d, 2 H, *J =* 2.5 Hz), 7.35 (d, 2 H, *J* = 2.5 Hz), 7.29 (s, 1 H), 6.78 (s, 1 H), 2.86 (q, 4 H, *J* = 10.6 Hz), 1.27 (s, 9 H), 0.91 (t, 6 H, *J* = 7 Hz); ESI MS m/z 337.4 [M+H]⁺.

### 5-Amino-2-cyclohexyl-6-(piperidin-1-yl)-1H-benzo[d]imidazole:

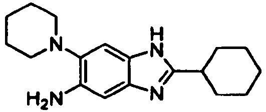

Yield 66 %; ¹HNMR (300 MHz, CDCl₃) δ 7.23 (s, 1 H), 6.81 (s, 1 H), 2.81 (t, 4 H, *J* = 4.9 Hz), 2.04 (m, 1 H), 1.78-1.23 (m, 16 H); ESI MS m/z 299.4 [M+H]⁺.

### EXAMPLES 35-48

The following key intermediates were prepared and characterized in the same manner as Examples 9-11.

### 2-Cyclohexyl-6-diethylamino-5-(4-methoxybenzoyl)amino-1H-[d]benzimidazole:

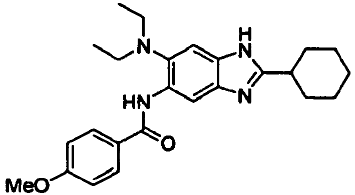

Yield 78%; ¹HNMR (300 MHz, CDCl₃) δ 10.31 (s, 1 H), 8.94 (s, 1 H), 7.95 (d, 2 H, *J* = 4.35 Hz), 7.57 (s, 1 H), 7.05 (d, 2 H, *J* = 4.35 Hz), 3.9 (s, 3 H), 3.0 (m, 4 H), 2.1 (s, 1 H), 1.98 (m, 2 H), 1.58 (m, 5 H), 1.26 (m, 3 H), 0.97 (t, 6 H, *J* = 7.2 Hz); ESI MS m/z 421.5 [M+H]⁺.

### 5-Benzoylamino-2-cyclohexyl-6-diethylamino-1H-benzo[d]imidazole:

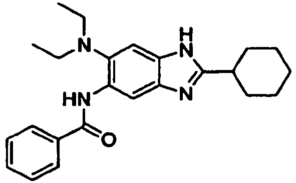

Yield 74 %; ¹HNMR (300 MHz, CDCl3) δ 10.3 (s, 1 H), 8.96 (s, 1 H), 7.98 (m, 2 H), 7.57 (m, 4 H), 3.03 (m, 4 H, *J* = 10.65 Hz), 1.98 (m, 2 H), 1.65 (m, 5 H), 1.16 (m, 4 H), .97 (m, 6 H, *J* = 7.2 Hz); ESI MS m/z 391.5 [M+H]⁺.

### 2-Cyclohexyl-6-diethylamino-5-(4-methylbenzoyl)amino-1H-benzo[d]imidazole:

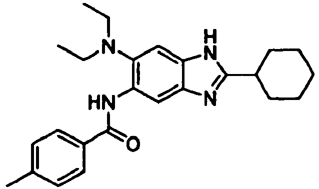

Yield 65 %; ¹HNMR (300 MHz, CDCl₃) δ 10.31 (s, I H), 8.91 (s, 1 H), 7.87 (d, 2 H, *J =* 4.5 Hz), 7.59 (s, 1 H), 7.34 (d, 2 H, *J* = 4.2 Hz), 3.02 (m, 4 H, *J* = 10.8 Hz), 2.45 (s, 3 H), 2.01 (m, 2 H), 1.69 (m, 5 H), 1.2 (m, 4 H), 0.97 (m, 6 H, *J* = 7 Hz) ESI MS m/z 405.5 [M+H]⁺.

### 5-(4-Chlorobenzoyl)amino-2-cyclohexyl-6-diethylamino-1H-benzo[d]imidazole:

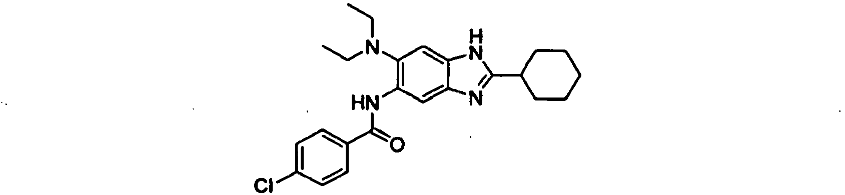

Yield 64 %; ¹HNMR (300 MHz, CDCl₃) δ 10.26 (s, I H), 8.77 (s, 1 H), 7.88 (d, 2 H, *J* = 3.45 Hz), 7.60 (s, 1 H), 7.50 (d, 2 H; *J=* 4.2 Hz), 3.02 (m, 4 H, *J =* 10.65 Hz), 2.11 (m, 2 H), 1.84-1.25(m, 9 H), 0.97 (t, 6 H, *J* = 7.2 Hz); ESI MS m/z 425 [M+H]⁺.

### 5-Benzyloxycarbonylamino-2-cyclohexyl-6-diethylamino-1H-benzo[d]imidazole:

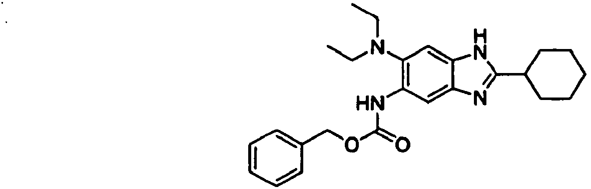

Yield 61 %; ¹HNMR (300 MHz, CDCl₃) δ 8.61 (s, 1 H), 8.25 (s, 1 H), 7.45-7.35 (m, 5 H), 5.22 (s, 2 H), 2.91 (m, 4 H, *J* = 10.65 Hz), 2.11 (m, 2 H), 1.84-1.62 (m, 5 H), 1.38 (m, 4 H), 0.90 (t, 6 H, *J* = 7.2 Hz); ESI MS m/z 421.5 [M+H]⁺.

### 2-Cyclohexyl-6-diethylamino-5-propoxycarbonylamino-1H-benzo[d]imidazole:

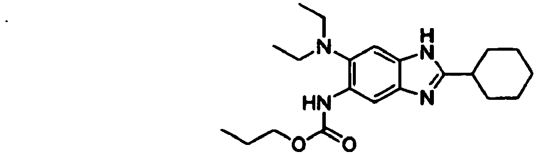

Yield 63 %; ¹HNMR (300 MHz, CDCl₃) δ 8.51 (s, 1 H), 8.23 (s, I H), 7.47 (s, 1 H), 4.14 (t, 2 H, *J* = 6.75 Hz), 2.92 (m, 4 H, *J* = 10.8 Hz), 2.10 (m, 2 H), 1.87-1.60 (m, 7 H), 1.40-1.25 (m, 4 H), 0.98 (t, 3 H, *J* = 6.15 Hz), 0.93 (t, 6 H, *J* = 7.05 Hz) ESI MS m/z 373.5 [M+H]⁺.

### 5-Butoxycarbonylamino-2-cyclohexyl-6-diethylamino-1H-benzo[d]imidazole:

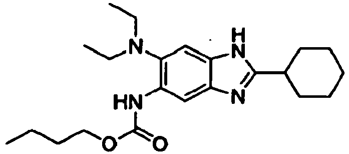

Yield 51 %; ¹HNMR (300 MHz, CDCl₃) δ 8.50 (s, 1 H), 8.22 (s, 1 H), 7.48 (s, 1 H), 4.18 (t, *J* = 6.75 Hz, 2 H), 2.92 (m, *J* = 10.5 Hz, 4 H), 2.10 (m, 2 H), 1.87-1.60 (m, 7 H), 1.40-1.39 (m, 6 H), 0.96 (t, *J* = 7.5 Hz, 3 H), 0.92 (t, *J* = 7.2 Hz, 6 H); ESI MS m/z 387.5 [M+H]⁺.

### 5-(But-3-enoxycarbonyl)amino-2-cyclohexyl-6-diethylamino-1H-benzo[d]imidazole:

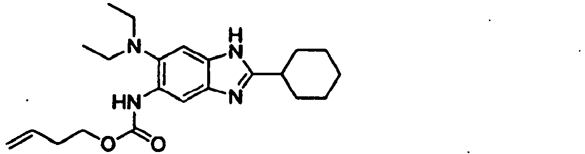

Yield 51 %; ¹HNMR (300 MHz, CDCl₃) δ 8.51 (s, I H), 8.23 (s, 1 H), 7.48 (s, 1 H), 5.84 (m, 1 H), 5.11 (m, 2 H), 4.23 (t, 2 H, *J* = 6.9 Hz), 2.92 (m, 4 H, *J* = 10.5 Hz), 2.47 (t, 2 H, *J* = 4.65 Hz), 2.10 (m, 2 H), 1.82-1.60 (m, 5 H), 1.40-1.32 (m, 4 H), 0.91 (t, 6 H, *J* = 7.05 Hz); ESI MS m/z 385.2 [M+H]⁺.

### 5-(4-tert-Butylbenzoylamino)-2-cyclohexyl-6-diethylamino-1H-benzo[d]imidazole:

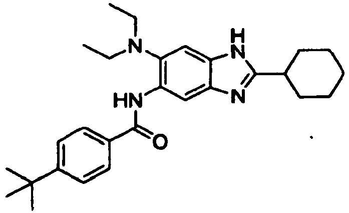

Yield 48 %; ¹HNMR (400 MHz, CDCl₃) δ 10.41 (s, 1 H), 9.02 (s, 1 H), 7.93 (d, 2 H, *J* = 4.2 Hz), 7.57 (d, 3 H, *J* = 4.2 Hz), 3.02 (q, 4 H, *J* = 10.6 Hz), 2.68 (m, 1 H), 1.92 (m, 2 H), 1.67-1.53 (m, 5 H), 1.38 (s, 9 H), 1.24 (s, 2 H), 1.26 (m, 3 H), 0.97 (t, 6 H, *J* = 7 Hz); ESI MS m/z 2447.6 [M+H]⁺.

### 2-Cyclohexyl-5-cyclopentanecarbonylamino-6-diethylamino-1H-benzo[d]imidazole:

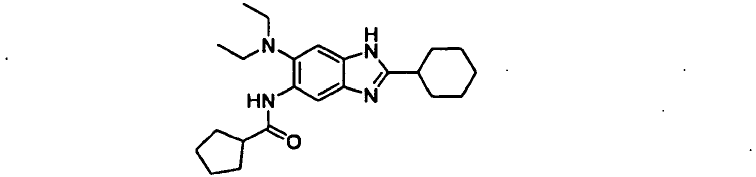

Yield 74 %; ¹HNMR (400 MHz, CDCl₃) δ 9.41 (s, 1 H), 8.65 (s, 1 H), 7.50 (s, 1 H), 2.93 (q, 4 H, *J* = 10.6 Hz), 2.81 (m, 2 H), 2.07-1.67 (m, 15 H), 1.31 (m, 3 H), 0.92 (t, 6 H, *J* = 7 Hz); ESI MS m/z 383.5 [M+H]⁺.

### 2-Cyclohexyl-6-diethylamino-5-(3-phenylpropanoyl)amino-1H-benzo[d]imidazole:

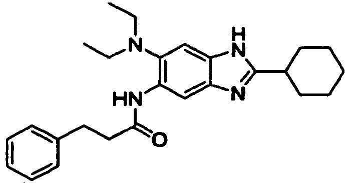

Yield 55 %; ¹HNMR (400 MHz, CDCl₃) δ 9.33 (s, 1 H), 8.69 (s, 1 H), 7.49 (s, 1 H), 7.59 (s, 1 H), 7.27-7.18 (m, 5 H), 3.11 (t, 2 H, *J* = 7.6 Hz), 2.86 (q, 3 H, *J* = 10.6 Hz), 2.76 (t, 2 H, *J* = 7.6 Hz), 2.08 (m, 2 H), 1.8-1.6 (m, 4 H), 1.35-1.23 (m, 5 H), 0.83 (t, 6 H, *J* = 7 Hz); ESI MS m/z 419.5 [M+H]⁺.

### 2-Cyclohexyl-6-diethylamino-5-pentanoylamino-1H-benzo[d]imidazole:

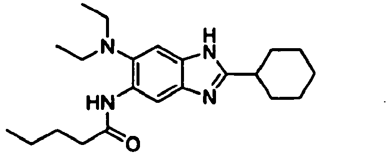

Yield 60 %; ¹HNMR (3\400 MHz, CDCl₃) δ 9.45 (s, 1 H), 8.73 (s, 1 H), 7.51 (s, 1 H), 2.94 (q, 4 H, *J* = 10.9 Hz), 2.84 (m, 1 H), 2.49 (t, 2 H, *J* = 7.6 Hz), 2.10 (m, 2 H), 1.84-1.65 (m, 7 H), 1.48-1.25 (m, 5 H), 0.96 (t, 3 H, *J* = 7.4 Hz), 0.92 (t, 6 H, *J* = 7.2 Hz); ESI MS m/z 371.5 [M+H]⁺.

### 5-Butanoylamino-2-cyclohexyl-6-diethylamino-1H-benzo[d]imidazole:

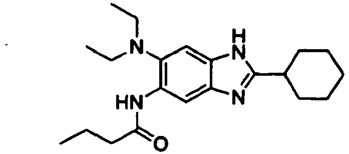

Yield 75 %; ¹HNMR (400 MHz, CDCl₃) δ 9.45 (s, 1 H), 8.73 (s, I H), 7.52 (s, 1 H), 2.94 (q, 4 H, *J* = 10.8 Hz), 2.84 (m, 1 H), 2.45 (t, 2 H, *J* = 7.6 Hz), 2.10 (m, 2 H), 1.84-1.65 (m, 7 H), 1.48-1.25 (m, 5 H), 1.06 (t, 3 H, *J =* 7.4 Hz), 0.92 (t, 6 H, *J* = 7.2 Hz); ESI MS m/z 357.5 [M+H]⁺.

### 2-Cyclohexyl-6-diethylamino-5-(prop-2-enoxycarbonyl)amino-1H-benzo[d]imidazole:

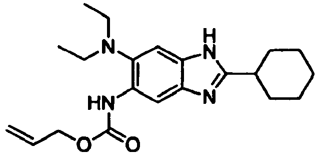

Yield 51 %; ¹HNMR (300 MHz, CDCl₃) δ 8.51 (s, 1 H), 8.23 (s, 1 H), 7.48 (s, 1 H), 5.84 (m, 1 H), 5.11 (m, 2 H), 4.23 (t, 2 H, *J* = 6.9 Hz), 2.92 (q, 4 H, *J* = 10.5 Hz), 2.47 (t, 2 H, *J* = 4.65 Hz), 2.10 (m, 2 H), 1.82-1.6 (m, 5 H), 1.40-1.32 (m, 4 H), 0.91 (t, 6 H, *J* = 7.05 Hz); ESI MS m/z 371.4 [M+H]⁺.

### PREPARATIVE EXAMPLES 49-54

The following key intermediates 49 through 54 were prepared and characterized in the same manner as 7-amino-5-(methoxycarbonyl)amino-2-(4-bromophenyl)-1H-benzo[d]imidazole in Example 12(b).

### 7-Amino-5-ethoxycarbonylamino-2-(furan-2-yl)-1H-benzo[d]imidazole:

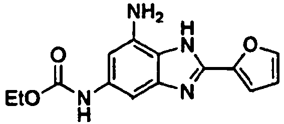

Yield 55%; ¹H-NMR (300MHz, CD₃OD) δ 1.29 (t, 3H, J = 9 Hz), 4.16 (dd, 2H, J = 14.1, 7.2 Hz), 6.51 (s, 1H), 6.61 (m, 1H), 7.05 (m, 1H), 7.13 (s, 1H), 7.67 (m, 1H); ESI MS m/z 287.0 [M+H]⁺.

### 7-Amino-5-methoxycarbonylamino-2-(4-methoxycarbonylphenyl)-1H-benzo[d]imidazole:

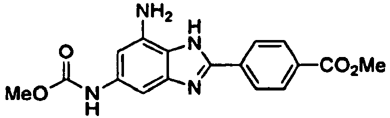

Yield 57%; ¹H-NMR (300MHz, CD₃OD) 8 3.73 (s, 3H), 3.93 (s, 3H), 6.52 (s, 1H), 7.19 (s, 1H), 8.12 (s, 4H); ESI MS m/z 341.0 [M+H]⁺.

### 7-Amino-5-ethoxylcarbonylamino-2-phenyl-1H-benzo[d]imidazole:

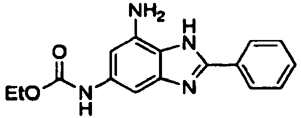

Yield 54%; ¹H-NMR (300MHz, CD₃OD) δ 1.30 (t, 3H, J = 6.9 Hz), 4.16 (dd, 2H, J = 14.4, 7.2 Hz), 6.51 (s, 1H), 7.18 (s, 1H), 7.44 (m, 3H), 8.01 (m, 2H); ESI MS m/z 297.1 [M+H]⁺.

### 7-Amino-2-(2,4-dimethoxyphenyl)-5-methoxycarbonylamino-1H-benzo[d]imidazole:

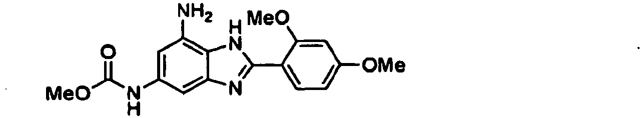

Yield 53%: ¹H-NMR (300MHz, CD₃OD) δ 3.72 (s, 3H), 3.85 (s, 3H), 3.99 (s, 3H), 6.51 (s, 1H), 6.66 (m, 2H), 7.17 (bs, 1H), 8.06 (d, 1H, J = 9.3 Hz); ESI MS m/z 343.0 [M+H]⁺.

### 7-Amino-2-(3-fluorophenyl)-5-methoxycarbonylamino-1H-benzo[d]imidazole:

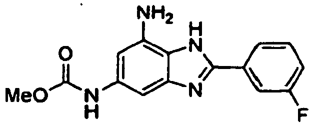

Yield 50%: ¹H-NMR (300MHz, CD₃OD) δ 3.72 (s, 3H), 6.51(s, 1H), 7.17 (m, 2H), 7.50 (m, 1H), 7.78 (m, 2H); ESI MS m/z 301.1 [M+H]⁺.

### 7-Amino-5-ethoxycarbonylamino-2-(phenylamino)-1H-benzo[d]imidazole:

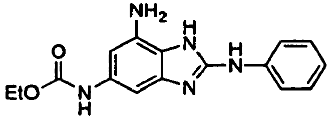

Yield 52%; ¹H-NMR (300MHz, CD₃OD) δ 130 (t, 3H, J = 7.2 Hz), 4.14 (dd, 2H, J = 14.1, 7.2 Hz), 6.48 (s, 1H), 6.95 (m, 1H), 7.27 (t, 2H), 7.43 (m, 2H ); ESI MS m/z 311.9 [M+H]⁺.

### EXAMPLES 55-56

**The following key intermediates 55 and 56 were prepared and characterized in the** same manner as **7-acetylamino-5-(methoxycarbonyl)amino-2-(4-bromophenyl)-1H-benzo[d]imidazole in Example** 12 (c).

### 7-Acetylamino-5-ethoxycarbonylamino-2-phenyl-1H-benzo[d]imidazole:

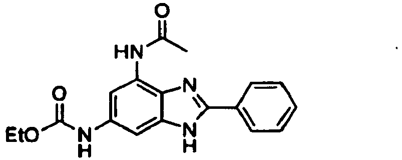

Yield 85%; ¹H-NMR (300MHz, CD₃OD) δ 1.30 (t, 3H, J = 7.2 Hz), 2.32 (s, 3H), 4.24 (dd, 2H, J = 14.4, 7.2 Hz), 7.58 (m, 3H), 7.77 (bs, 1H), 7.85 (bs, 1H), 8.10 (m, 2H); ESI MS m/z 339.1 [M+H]⁺.

### 7-Acetylamino-2-(3-fluorophenyl)-5-methoxycarbonylamino-1H-benzo[d]imidazole:

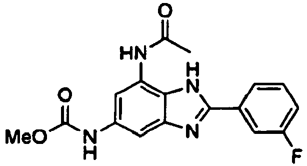

Yield 83%: ¹H-NMR (300MHz, CD₃OD) δ 2.25 (s, 3H), 3.74 (s, 3H), 6.51(s, 1H), 7.22 (m, 1H), 7.52 (m, 1H), 7.78 (m, 5H); ESI MS m/z 343.1 [M+H]⁺.

### APPENDIX

### Active benzimidazole derivatives

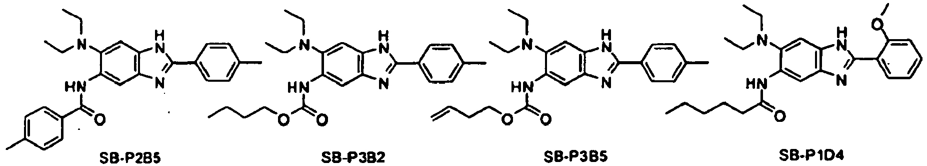

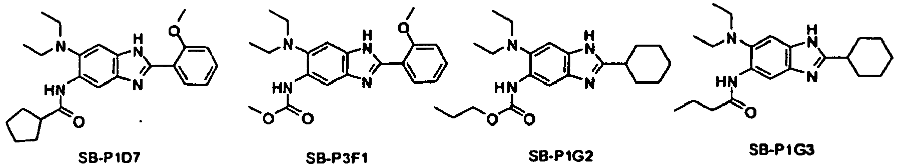

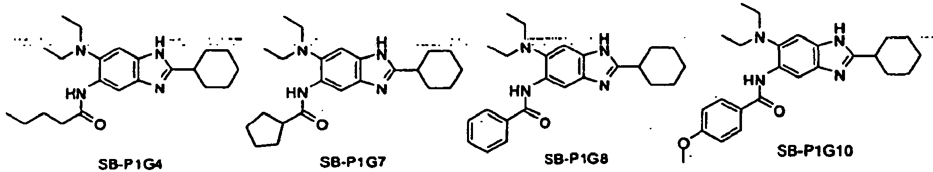

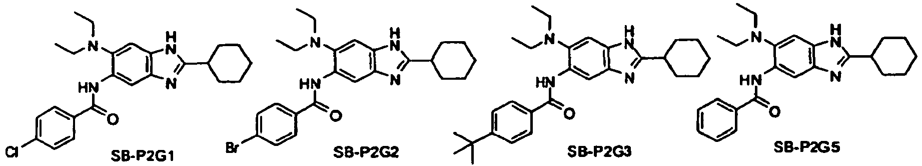

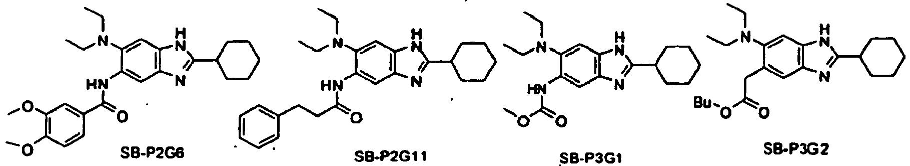

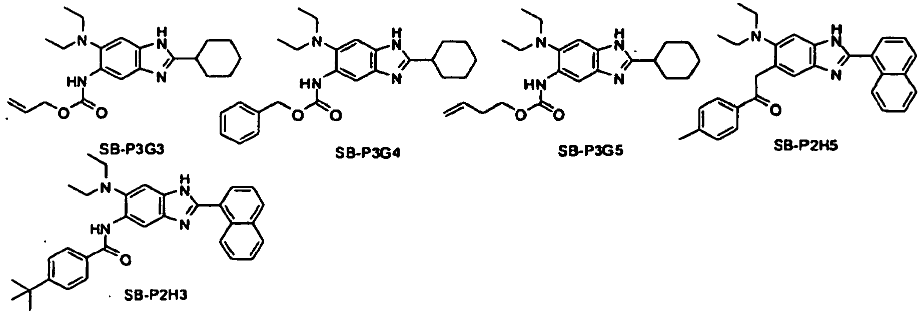

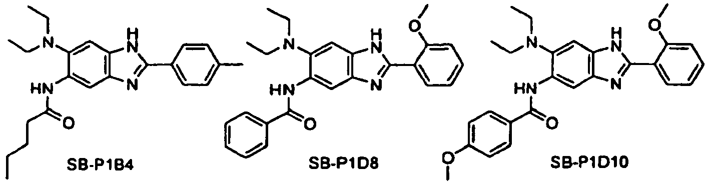

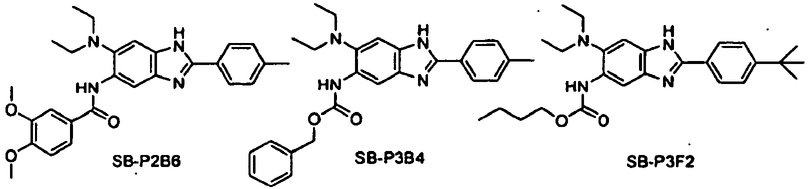

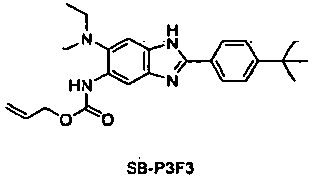

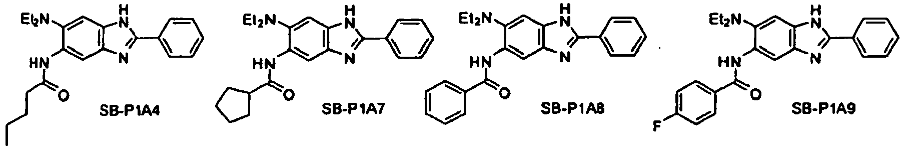

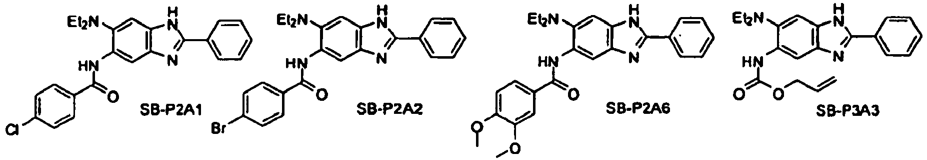

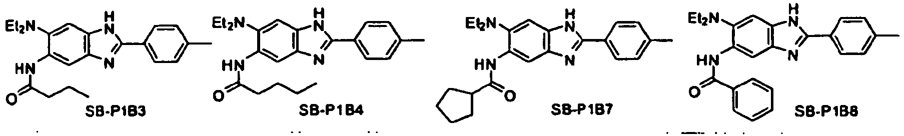

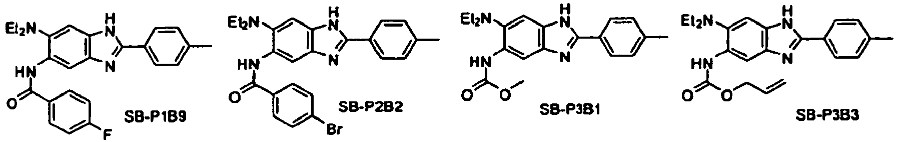

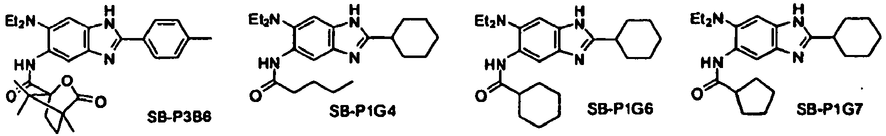

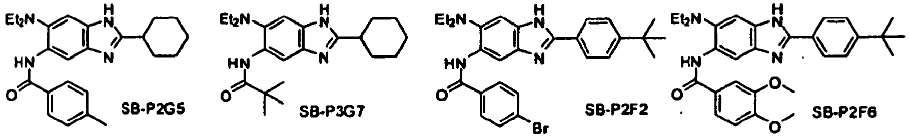

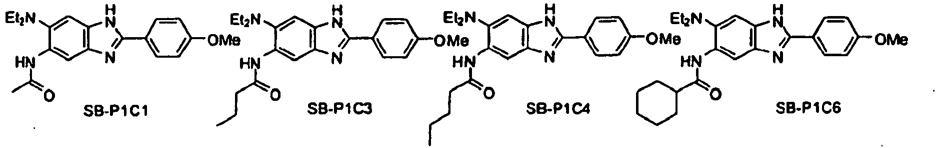

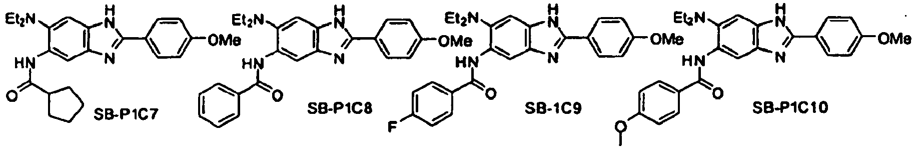

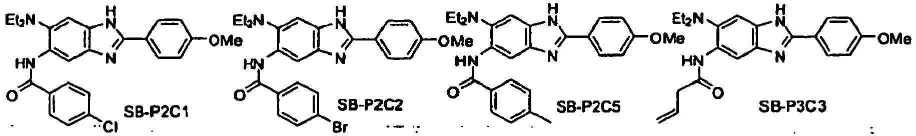

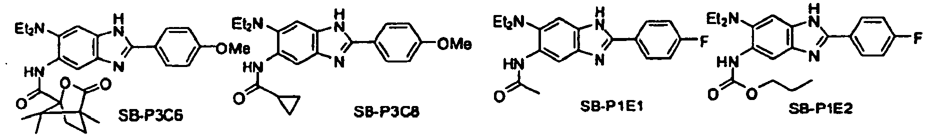

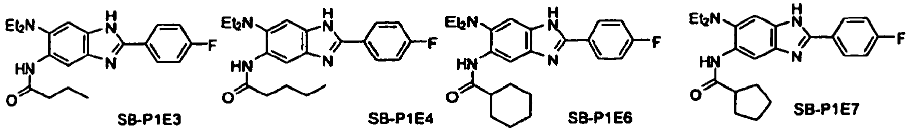

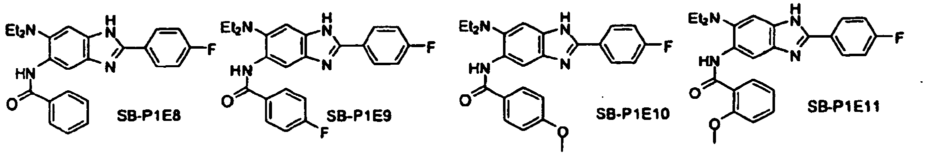

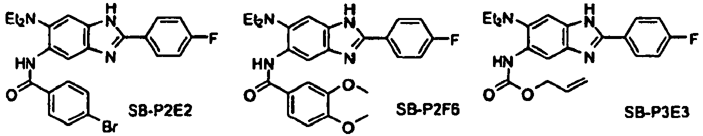

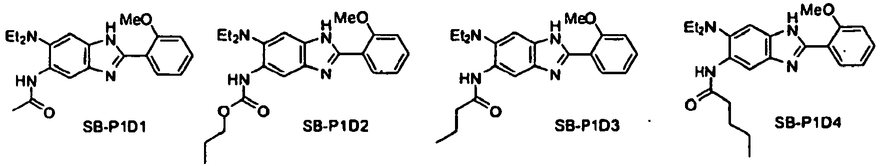

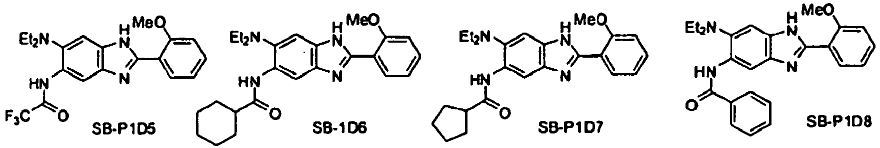

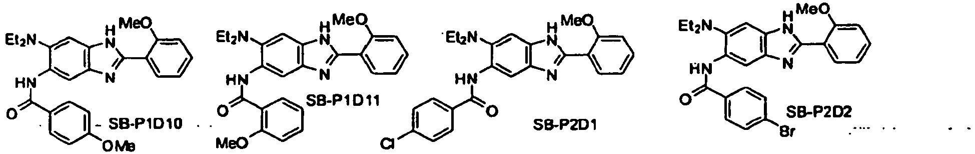

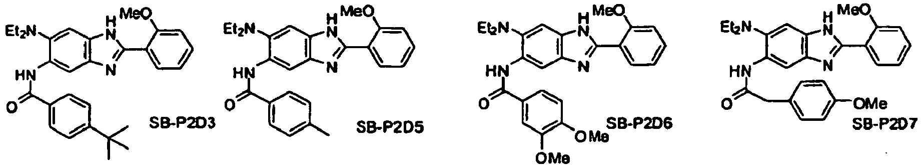

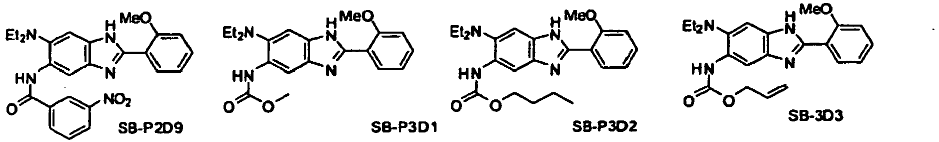

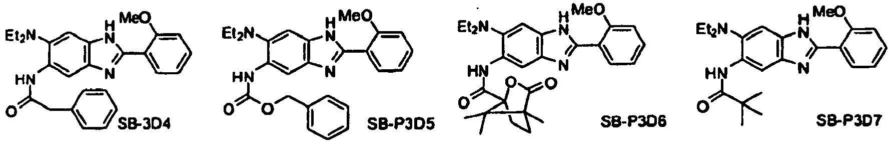

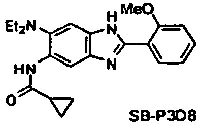

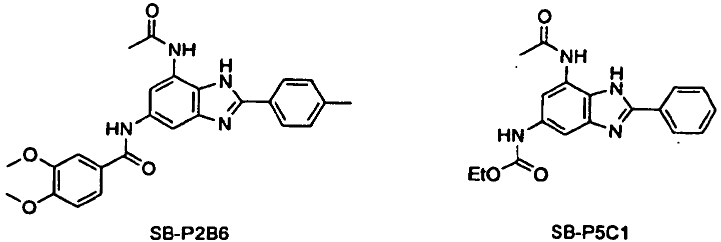

## Claims

1. A molecule having the formula I 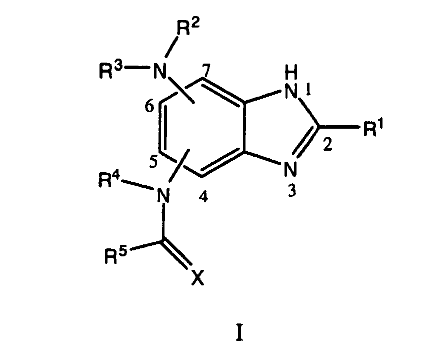 wherein:
R¹ represents NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, alkyl, cycloalkyl, aryl, or halo;
R² and R⁴ independently represent H, alkyl, cycloalkyl, or aryl;
R³ represents alkyl, cycloalkyl, or aryl;
R⁵ represents H, R⁶, OR⁶, SR⁶, NH₂, NHR⁶, or NR⁹R¹⁰;
X represents O, S, NH, or NR⁶;
R⁶, R⁷, R⁸, R⁹, and R¹⁰ independently represent alkyl, cycloalkyl, aryl, or halo;
R² and R³; R⁴ and R⁵; and R⁹ and R¹⁰ independently, may be combined to represent a heterocyclic alkyl or heterocyclic aryl;
R⁷ and R⁸ may be combined to represent a cycloalkyl;
alkyl groups are branched or unbranched, saturated or unsaturated, and have 1-18 carbon atoms in their longest chain;
cycloalkyl groups are carbocyclic or heterocyclic, fused or unfused, non-aromatic ring systems having a total of 5-16 ring members including substituent rings;
aryl groups are carbocyclic or heterocyclic;
carbocyclic aryl groups are fused or unfused ring systems having a total of 6-16 ring members including substituent rings;
heterocyclic aryl groups are fused or unfused ring systems having a total of 5-16 ring members including substituent rings;
halo substituents are fluoro, chloro, or bromo;
each alkyl, cycloalkyl, and aryl, independently, may be unsubstituted or substituted with one or more substituent at any position;
alkyl substituents are halo, hydroxyl, OR⁶, SR⁶, NH₂, NHR⁶, NR⁹R¹⁰, cycloalkyl, or aryl;
cycloalkyl substituents are halo, hydroxyl, OR⁶, SR⁶, NH2, NHR⁶, NR⁹R¹⁰, alkyl, cycloalkyl, or aryl;
aryl substituents are halo, hydroxyl, OR⁶, SR⁶, NH₂, NHR⁶, NR⁹R¹⁰, alkyl, cycloalkyl, aryl, nitro, or carboxyl; and
heterocyclic alkyl and heterocyclic aryl have at least one heteroatom selected from oxygen, nitrogen and sulfur; and
pharmaceutically acceptable salts thereof.

2. A molecule according to claim 1, wherein:
R¹ represents cycloalkyl or aryl;
R² and R³ independently represent C1-C4 alkyl.

3. A molecule according to claim 2, wherein:
R⁴ is H; and
X is O.

4. A molecule according to claim 2, wherein:
R¹ represents 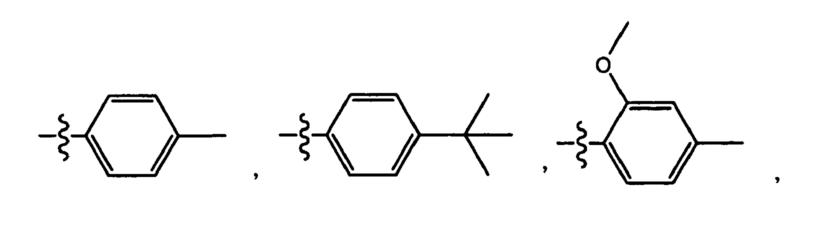 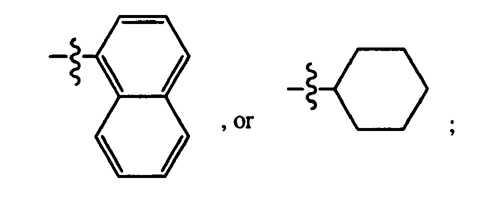
R² and R³ represent ethyl;
R⁴ represents H;
R⁵ represents 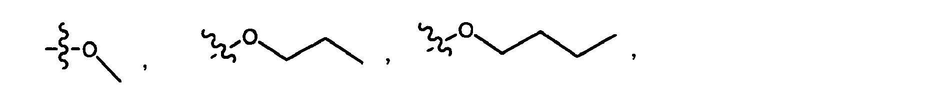 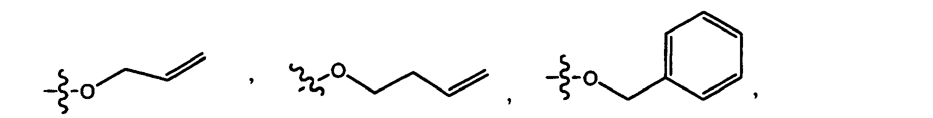 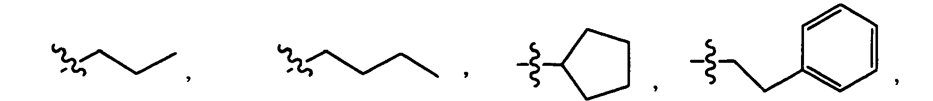 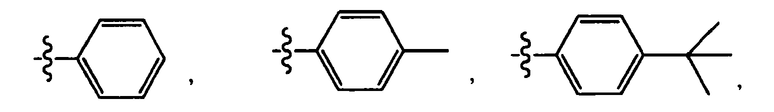 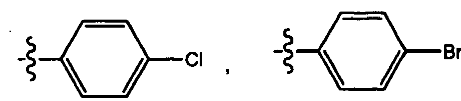 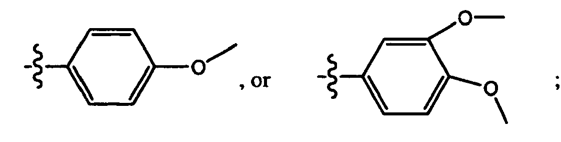 and
X represents O.

5. A molecule according to claim 2, wherein:
R¹ represents 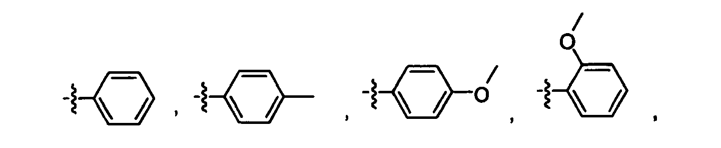 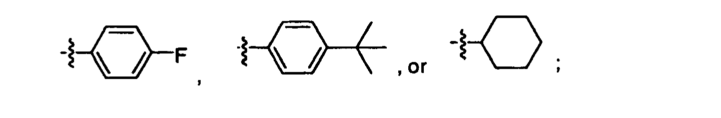
R² and R³ represent ethyl;
R⁴ represents H;
R⁵ represents 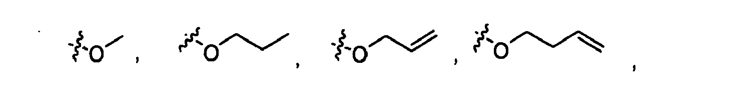 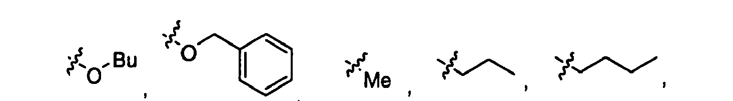 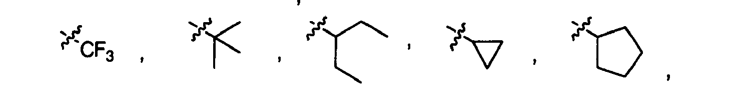 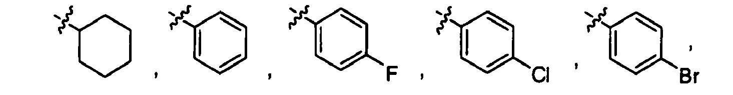 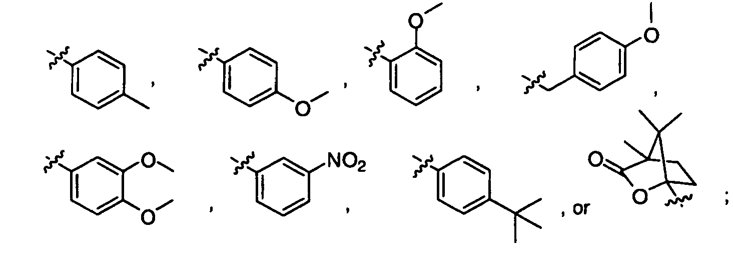 and
X represents O.

6. A molecule according to claim 5, wherein:
(i) R¹ represents 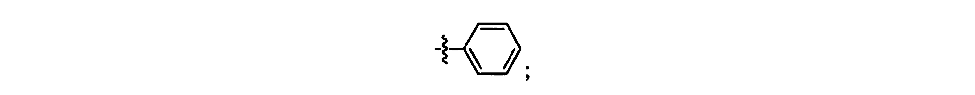 and
R⁵ represents 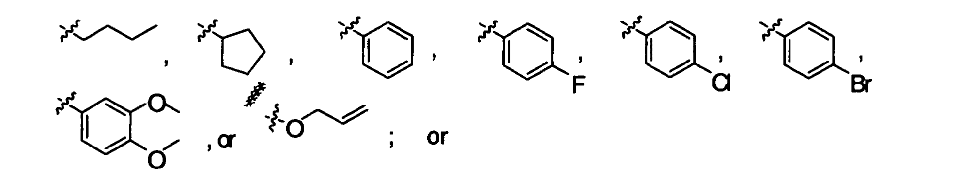
(ii) R¹ represents 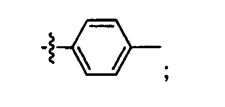 and
R⁵ represents 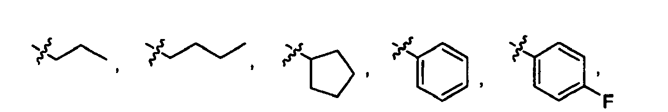 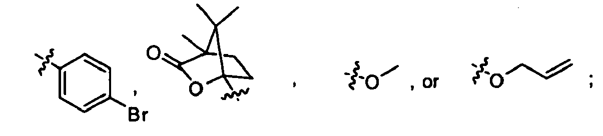 or
(iii) R¹ represents 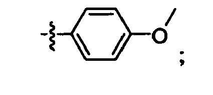 and
R⁵ represents 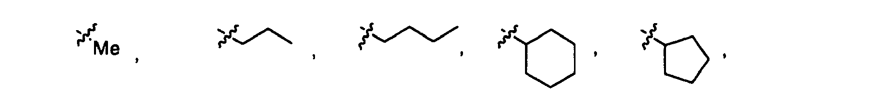 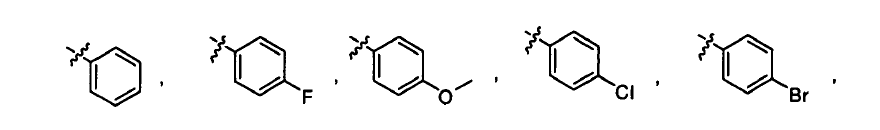 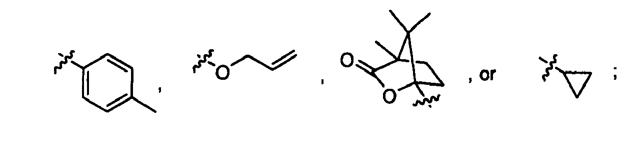 or
(iv) R¹ represents 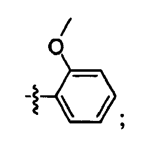 and
R⁵ represents 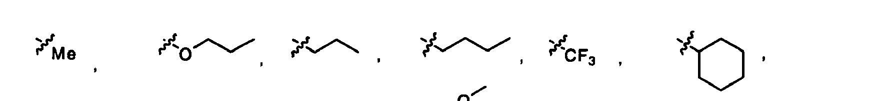 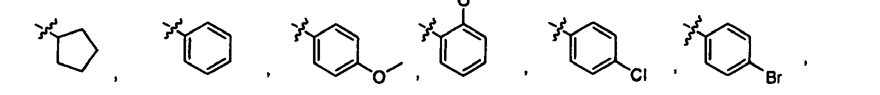 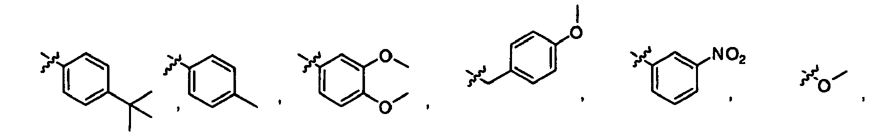 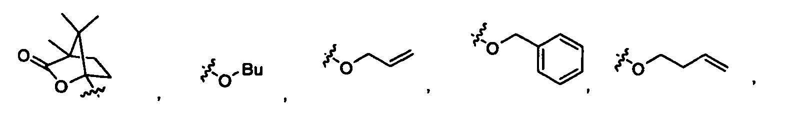 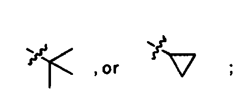 or
(v) R¹ represents 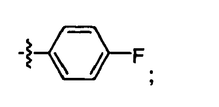 and
R⁵ represents 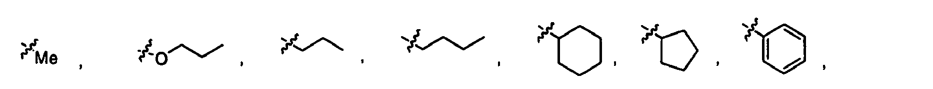 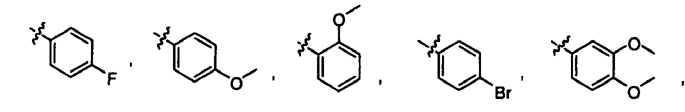 or 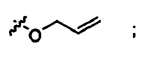 or
(vi) R¹ represents 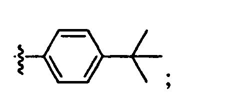 and
R⁵ represents 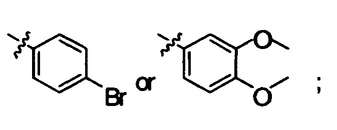 or
(vii) R¹ represents 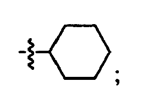 and
R⁵ represents

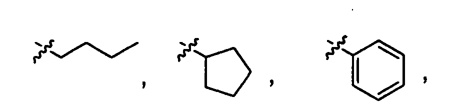

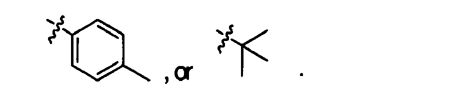

7. A molecule according to claim 1, wherein:
when R² represents H, R³ is not methyl.

8. A compound of formula I or pharmaceutically acceptable salts thereof as defined in claim 1 for use in treating a patient infected with *Mycobacterium tuberculosis.* I

9. A compound of formula I or pharmaceutically acceptable salts thereof as defined in claim 1 for use in treating a patient infected with *Francisella tulerensis.*

10. A compound or pharmaceutically acceptable salt according to claim 1 or for use according to any one of claims 8 or 9, wherein:
R⁶, R⁷, R⁸, R⁹, and R¹⁰ independently represent alkyl, cycloalkyl, or aryl.

11. A molecule having the formula I 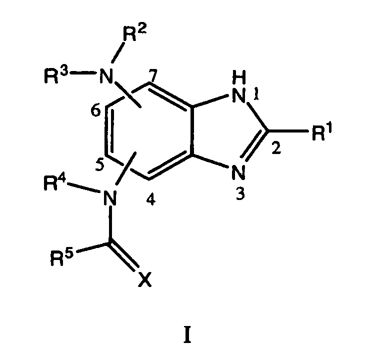 wherein:
R¹ represents NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, alkyl, cycloalkyl, aryl, or halo;
R² and R⁴ independently represent H, alkyl, cycloalkyl, or aryl;
R⁵ represents H, R⁶, OR⁶, SR⁶, NH₂, NHR⁶, or NR⁹R¹⁰;
R⁶, R⁷, R⁸, R⁹, and R¹⁰ independently represent alkyl, cycloalkyl, or aryl;
R² and R³; R⁴ and R⁵; and R⁹ and R¹⁰ independently, may be combined to represent a heterocyclic alkyl or heterocyclic aryl;
R⁷ and R⁸ may be combined to represent a cycloalkyl;
and wherein:
X represents O; and
R³ represents alkyl, cycloalkyl, aryl, CO(cycloalkyl) or CO(cycloaryl);
or
X represents S, NH, or NR⁶; and
R³ represents alkyl, cycloalkyl, aryl, or COR⁶;
or
X represents O;
R³ represents COCH₃; and
R¹, R², R⁴, R⁵ and R⁶ are as shown in the compounds of formula I below: 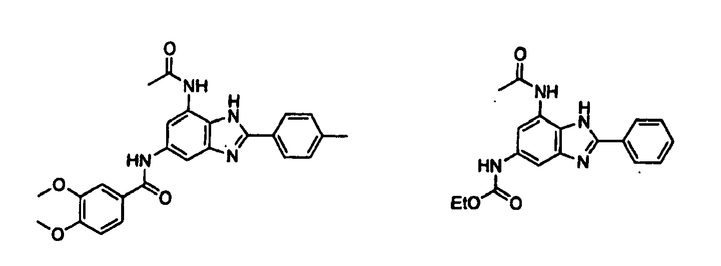 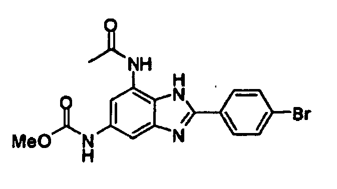 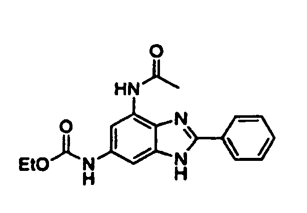 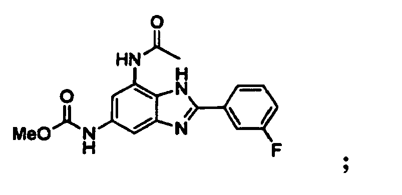
and wherein:
alkyl groups are branched or unbranched, saturated or unsaturated, and have 1-18 carbon atoms in their longest chain;
cycloalkyl groups are carbocyclic or heterocyclic, fused or unfused, non-aromatic ring systems having a total of 5-16 ring members including substituent rings;
aryl groups are carbocyclic or heterocyclic;
carbocyclic aryl groups are fused or unfused ring systems having a total of 6-16 ring members including substituent rings;
heterocyclic aryl groups are fused or unfused ring systems having a total of 5-16 ring members including substituent rings;
halo substituents are fluoro, chloro, or bromo;
each alkyl, cycloalkyl, and aryl, independently, may be unsubstituted or substituted with one or more substituent at any position;
alkyl substituents are halo, hydroxyl, OR⁶, SR⁶, NH₂, NHR⁶, NR⁹R¹⁰, cycloalkyl, or aryl;
cycloalkyl substituents are halo, hydroxyl, OR⁶, SR⁶, NH₂, NHR⁶, NR⁹R¹⁰, alkyl, cycloalkyl, or aryl;
aryl substituents are halo, hydroxyl, OR⁶, SR⁶, NH₂, NHR⁶, NR⁹R¹⁰, alkyl, cycloalkyl, aryl, nitro, or carboxyl; and
heterocyclic alkyl and heterocyclic aryl have at least one heteroatom selected from oxygen, nitrogen and sulfur; and
pharmaceutically acceptable salts thereof.

12. A compound of formula I or pharmaceutically acceptable salts thereof as defined in claim 11 for use in treating a patient infected with *Mycobacterium tuberculosis.*

13. A compound of formula I or pharmaceutically acceptable salts thereof as defined in claim 11 for use in treating a patient infected with *Francisella tulerensis.*

## Patentansprüche

1. Molekül der Formel I 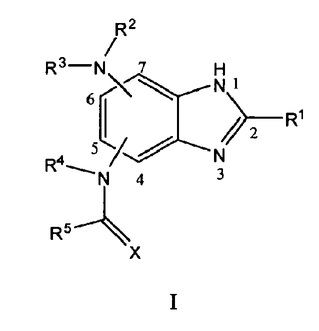 wobei:
R¹ für NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, Alkyl, Cycloalkyl, Aryl oder Halogen steht;
R² und R⁴ unabhängig voneinander für H, Alkyl, Cycloalkyl oder Aryl stehen;
R³ für Alkyl, Cycloalkyl oder Aryl steht;
R5 für H, R⁶, OR⁶, SR⁶, NH₂, NHR⁶, oder NR⁹R¹⁰ steht;
X für O, S, NH oder NR⁶ steht;
R⁶, R⁷, R⁸, R⁹ und R¹⁰ unabhängig voneinander für Alkyl, Cycloalkyl, Aryl oder Halogen stehen;
R² und R³; R⁴ und R⁵; und R⁹ und R¹⁰ unabhängig voneinander so kombiniert werden können, dass sie für ein heterozyklisches Alkyl oder heterozyklisches Aryl stehen;
R⁷ und R⁸ so kombiniert werden können, dass sie für ein Cycloalkyl stehen;
Alkylgruppen verzweigt oder unverzweigt, gesättigt oder ungesättigt sind, und 1-18 Kohlenstoffatomen in ihrer längsten Kette aufweisen;
Cycloalkylgruppen carbozyklische oder heterozyklische, kondensierte oder nichtkondensierte, nichtaromatische Ringsysteme mit insgesamt 5-16 Ringgliedern einschließlich Substituentenringen sind;
Arylgruppen carbozyklisch oder heterozyklisch sind;
carbozyklische Arylgruppen kondensierte oder nichtkondensierte Ringsysteme mit insgesamt 6-16 Ringgliedern einschließlich Substituentenringen sind;
heterozyklische Arylgruppen kondensierte oder nichtkondensierte Ringsysteme mit insgesamt 5-16 Ringgliedern einschließlich Substituentenringen sind;
Halogensubstituenten Fluor, Chlor, oder Brom sind;
Alkyl, Cycloalkyl und Aryl jeweils unabhängig voneinander unsubstituiert oder substituiert mit einem oder mehreren Substituenten an jeder Position sein können;
Alkylsubstituenten Halogen, Hydroxyl, OR⁶, SR⁶, NH₂, NHR⁶, NR⁹R¹⁰, Cycloalkyl, oder Aryl sind;
Cycloalkylsubstituenten Halogen, Hydroxyl, OR⁶, SR⁶, NH₂, NHR⁶, NR⁹R¹⁰, Alkyl, Cycloalkyl, oder Aryl sind;
Arylsubstituenten Halogen, Hydroxyl, OR⁶, SR⁶, NH₂, NHR⁶, NR⁹R¹⁰, Alkyl, Cycloalkyl, Aryl, Nitro oder Carboxyl sind; und
heterozyklisches Alkyl und heterozyklisches Aryl mindestens ein Heteroatom ausgewählt aus Sauerstoff, Stickstoff und Schwefel aufweisen; und
pharmazeutisch akzeptable Salze davon.

2. Molekül nach Anspruch 1, wobei:
R¹ für Cycloalkyl oder Aryl steht;
R² und R³ unabhängig voneinander für C₁-C₄-Alkyl stehen.

3. Molekül nach Anspruch 2, wobei:
R⁴ H ist; und
X O ist.

4. Molekül nach Anspruch 2, wobei:
R¹ für 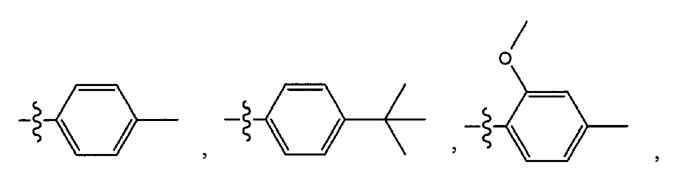 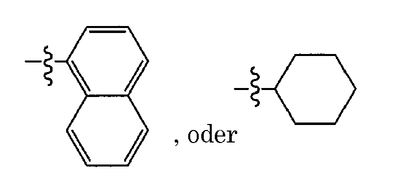 steht;
R² und R³ für Ethyl stehen;
R4 für H steht;
R⁵ für 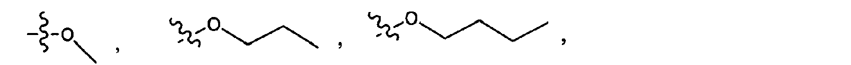 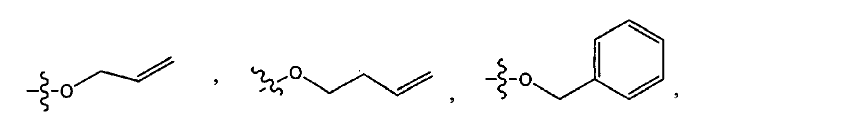 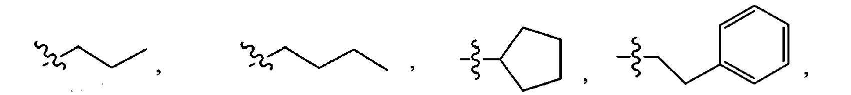 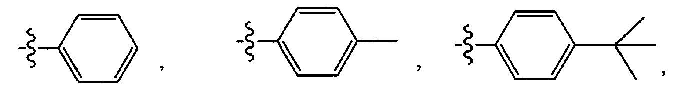 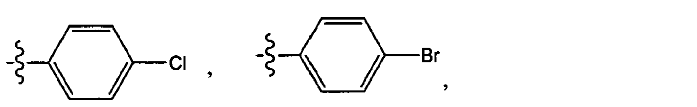 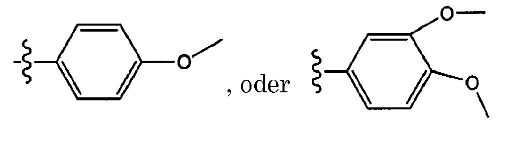 steht; und
X für O steht.

5. Molekül nach Anspruch 2, wobei:
R¹ für 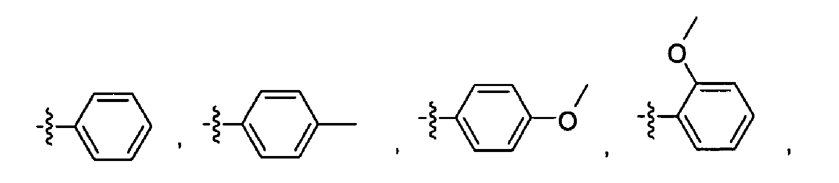 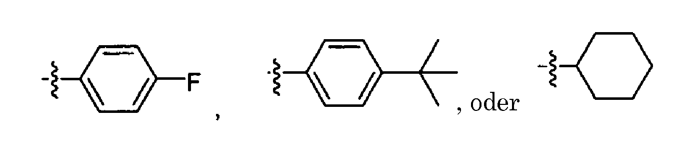 steht;
R² und R³ für Ethyl stehen;
R⁴ für H steht;
R⁵ für 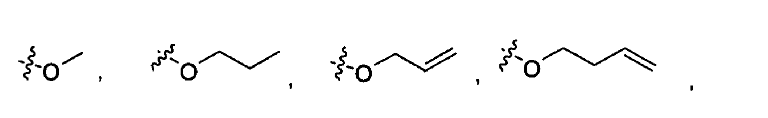 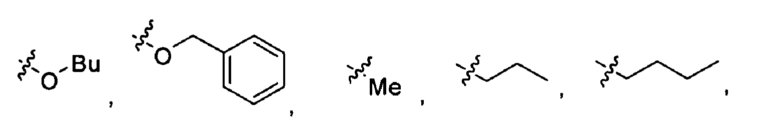 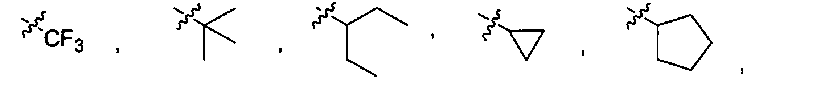 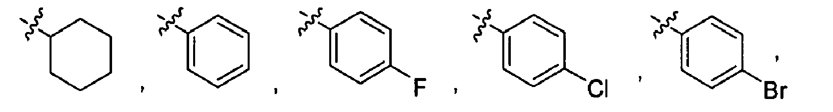 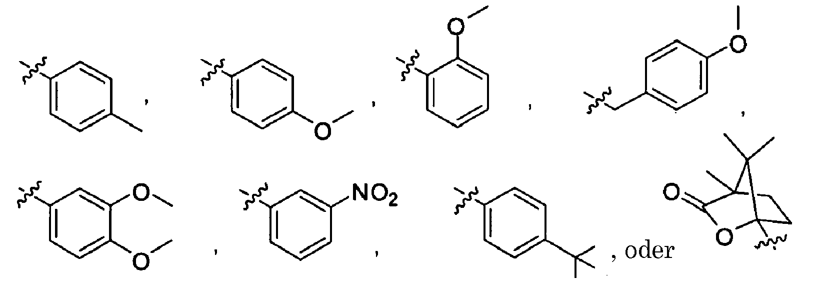 steht;
und
X für O steht.

6. Molekül nach Anspruch 5, wobei:
(i) R¹ für 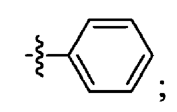 steht
und R⁵ für 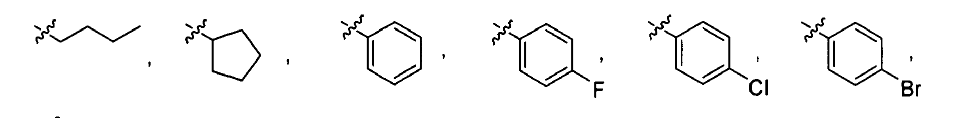 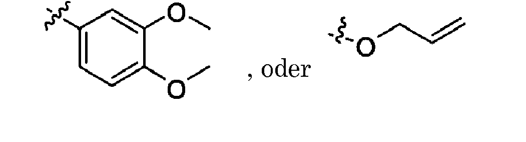 steht; oder
(ii) R¹ für 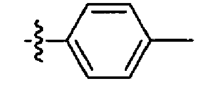 steht; und
R⁵ für 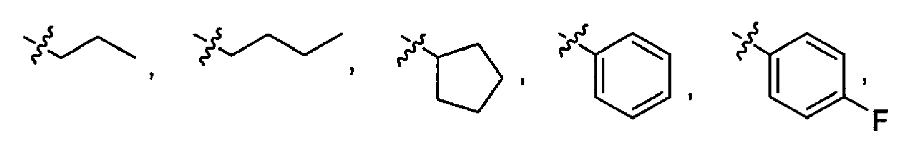 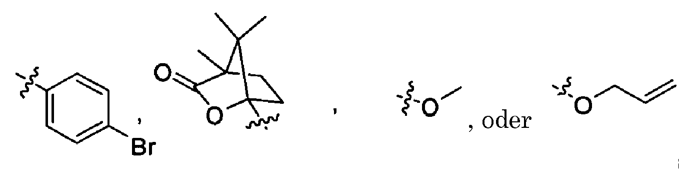 steht; oder
(iii) R¹ für 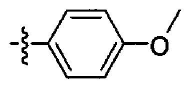 steht; und
R⁵ für 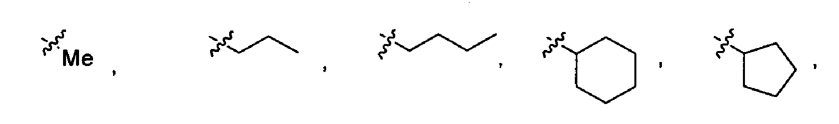 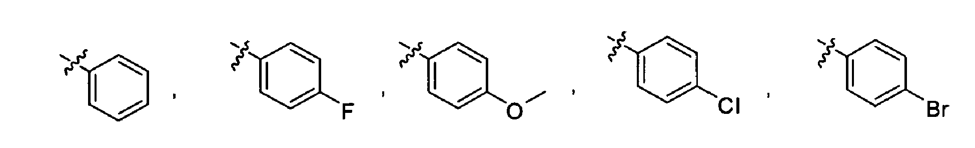 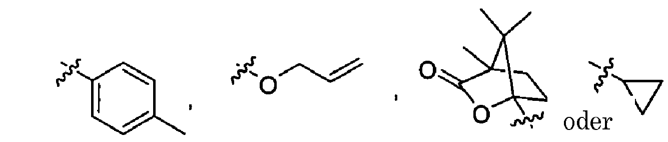 steht; oder
(iv) R¹ für 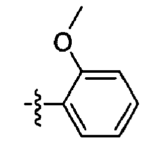 steht; und
R⁵ für 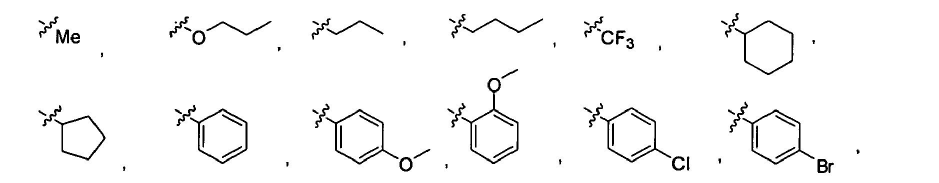 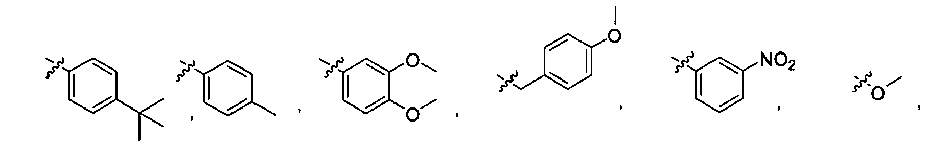 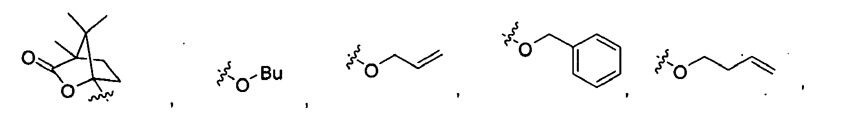 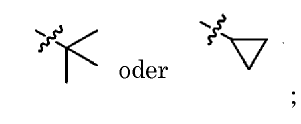 oder
(v) R¹ für 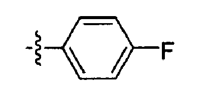 steht; und
R⁵ für 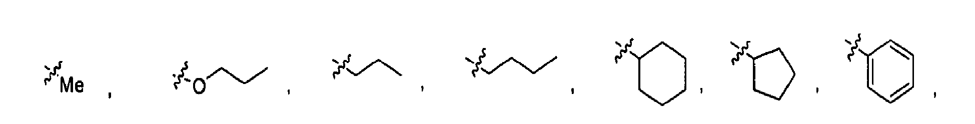 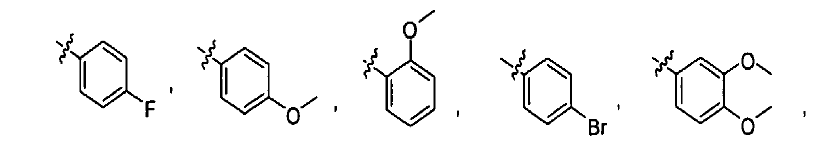 oder 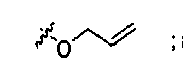 oder
(vi) R¹ für 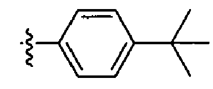 steht; und
R⁵ für 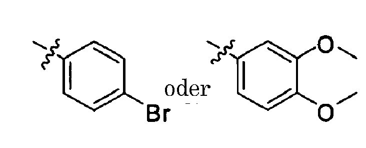 steht; oder
(vii) R¹ für 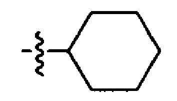 steht; und
R⁵ für 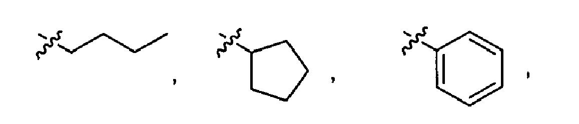 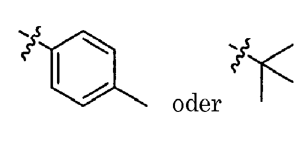 steht.

7. Molekül nach Anspruch 1, wobei:
wenn R² für H steht, dann ist R³ nicht Methyl.

8. Verbindung der Formel I oder ein pharmazeutisch akzeptables Salz davon wie in Anspruch 1 definiert, zur Verwendung in der Behandlung eines mit *Mycobacterium tuberculosis* infizierten Patienten.

9. Verbindung der Formel I oder ein pharmazeutisch akzeptables Salz davon wie in Anspruch 1 definiert, zur Verwendung in der Behandlung eines mit *Francisella tulerensis* infizierten Patienten.

10. Verbindung oder pharmazeutisch akzeptables Salz nach Anspruch 1 oder zur Verwendung nach einem der Ansprüche 8 oder 9, wobei:
R6, R⁷, R⁸, R⁹, und R¹⁰ unabhängig voneinander für Alkyl, Cycloalkyl, oder Aryl stehen.

11. Molekül der Formel I 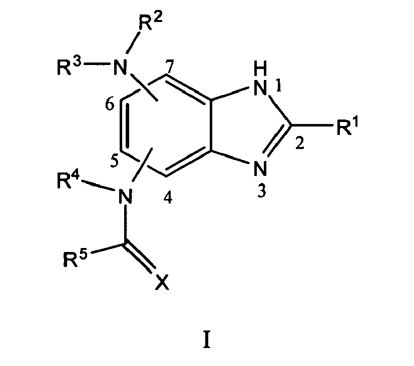 wobei:
R¹ für NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, Alkyl, Cycloalkyl, Aryl oder Halogen steht;
R² und R⁴ unabhängig voneinander für H, Alkyl, Cycloalkyl oder Aryl stehen;
R⁵ für H, R⁶, OR⁶, SR⁶, NH₂, NHR⁶ oder NR⁹R¹⁰ steht;
R⁶, R⁷, R⁸, R⁹ und R¹⁰ unabhängig voneinander für Alkyl, Cycloalkyl, oder Aryl stehen;
R² und R³, R⁴ und R⁵; und R⁹ und R¹⁰ unabhängig so kombiniert werden können, dass sie für ein heterozyklisches Alkyl oder heterozyklisches Aryl stehen;
R⁷ und R⁸ so kombiniert werden können, dass sie für eine Cycloalkylgruppe stehen;
und wobei:
X für O steht; und
R³ für Alkyl, Cycloalkyl, Aryl, CO(cycloalkyl) oder CO(cycloaryl) steht; oder
X für S, NH oder NR⁶ steht; und
R³ für Alkyl, Cycloalkyl, Aryl, oder COR⁶ steht, oder
X für O steht;
R³ für COCH³ steht; und
R¹, R², R⁴, R⁵ und R⁶ wie unten in den Verbindungen von Formel I gezeigt sind: 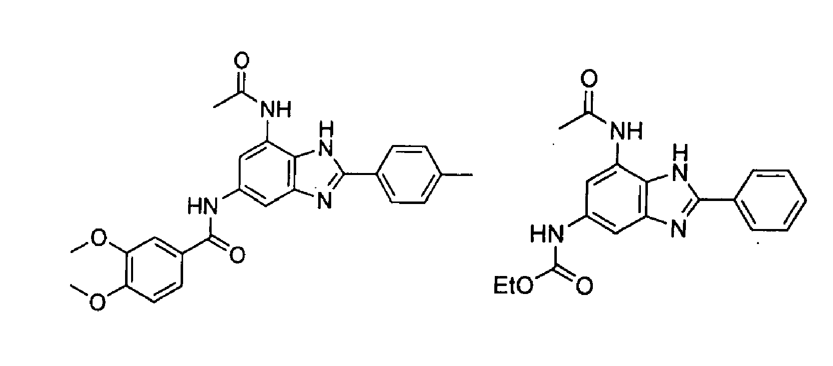 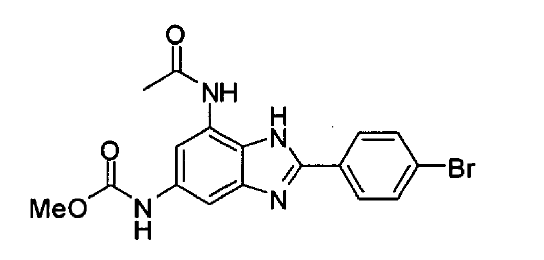 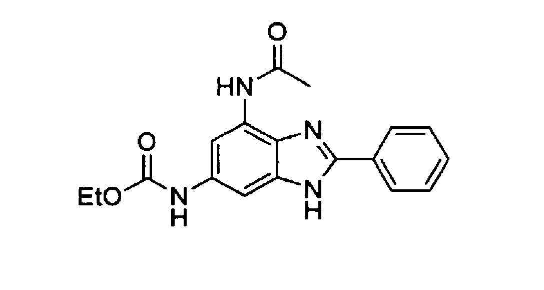 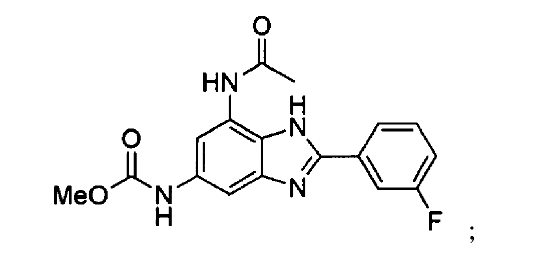
und wobei:
Alkylgruppen verzweigt oder unverzweigt, gesättigt oder ungesättigt sind und 1-18 Kohlenstoffatome in ihrer längsten Kette aufweisen;
Cycloalkylgruppen carbozyklische oder heterozyklische, kondensierte oder nichtkondensierte, nicht aromatische Ringsysteme mit insgesamt 5-16 Ringgliedern einschließlich Substituentenringen sind;
Arylgruppen carbozyklisch oder heterozyklisch sind;
carbozyklische Arylgruppen kondensierte oder nichtkondensierte Ringsysteme mit insgesamt 6-16 Ringgliedern einschließlich Substituentenringen sind;
heteroczyklische Arylgruppen kondensierte oder nichtkondensierte Ringsysteme mit insgesamt 5-16 Ringgliedern einschließlich Substituentenringen sind;
Halogensubstituenten Fluor, Chlor, oder Brom sind;
jedes Alkyl, Cycloalkyl und Aryl unabhängig voneinander unsubstituiert oder substituiert mit einem oder mehreren Substituenten an jeder Position ist; Alkylsubstituenten Halogen, Hydroxyl, OR⁶, SR⁶, NH₂, NHR⁶, NR⁹R¹⁰, Cycloalkyl, oder Aryl sind;
Cycloalkylsubstituenten Halogen, Hydroxyl, OR⁶, SR⁶, NH₂, NHR⁶, NR⁹R¹⁰, Alkyl, Cycloalkyl, oder Aryl sind;
Arylsubstituenten Halogen, Hydroxyl, OR⁶, SR⁶, NH₂, NHR⁶, NR⁹R¹⁰, Alkyl-, Cycloalkyl, Aryl, Nitro oder Carboxyl sind; und
heterozyklisches Alkyl und heterozyklisches Aryl mindestens ein Heteroatom ausgewählt aus Sauerstoff, Stickstoff und Schwefel aufweist; und pharmazeutisch akzeptable Salze davon.

12. Verbindung der Formel I oder pharmazeutisch akzeptable Salze davon wie in Anspruch 11 definiert, zur Verwendung in der Behandlung eines mit *Mycobacterium tuberculosis* infizierten Patienten.

13. Verbindung der Formel I oder pharmazeutisch akzeptable Salze davon wie in Anspruch 11 definiert, zur Verwendung in der Behandlung eines mit *Francisella tulerensis* infizierten Patienten.

## Revendications

1. Molécule ayant la formule I 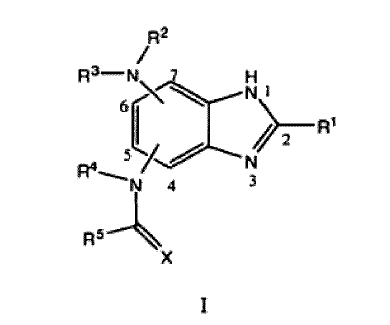 où :
R¹ représente NH₂, NHR⁶, NR⁹R¹⁰, NR⁶CONR⁹R¹⁰, NR⁶C⁵NR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, alkyle, cycloalkyle, aryle ou halo ;
R² et R⁴ représentent indépendamment H, alkyle, cycloalkyle ou aryle ;
R³ représente alkyle, cycloalkyle ou aryle ;
R⁵ représente H, R⁶, OR⁶, SR⁶, NH₂, NHR⁶ ou NR⁹R¹⁰ ;
X représente O, S, NH ou NR⁶ ;
R⁶, R⁷, R⁸, R⁹ et R¹⁰ représentent indépendamment alkyle, cycloalkyle, aryle ou halo ;
R² et R³ ; R⁴ et R⁵ ; et R⁹ et R¹⁰ indépendamment, peuvent être combinés pour représenter un alkyle hétérocyclique ou un aryle hétérocyclique;
R⁷ et R⁸ peuvent être combinés pour représenter un cycloalkyle ;
les groupes alkyle sont ramifiés ou non ramifiés, saturés ou insaturés, et ont 1-18 atomes de carbone dans leur chaîne la plus longue ;
les groupes cycloalkyle sont des systèmes cycliques, carbocycliques ou hétérocycliques, condensés ou non condensés, non aromatiques ayant un total de 5-16 chaînons cycliques incluant les cycles substituants ;
les groupes aryle sont carbocycliques ou hétérocycliques ;
les groupes aryle carbocycliques sont des systèmes cycliques condensés ou non condensés ayant un total de 6-16 chaînons cycliques incluant les cycles substituants ;
les groupes aryle hétérocycliques sont des systèmes cycliques condensés ou non condensés ayant un total de 5-16 chaînons cycliques incluant les cycles substituants ;
les substituants halo sont fluoro, chloro ou bromo ;
chaque alkyle, cycloalkyle et aryle, indépendamment, peut être non substitué ou substitué avec un ou plusieurs substituants à une position quelconque ;
les substituants d'alkyle sont halo, hydroxyle, OR⁶, SR⁶, NH₂, NHR⁶, NR⁹R¹⁰, cycloalkyle ou aryle ;
les substituants de cycloalkyle sont halo, hydroxyle, OR⁶, SR⁶, NH₂, NHR⁶, NR⁹R¹⁰, alkyle, cycloalkyle ou aryle ;
les substituants d'aryle sont halo, hydroxyle, OR⁶, SR⁶, NH₂, NHR⁶, NR⁹R¹⁰, alkyle, cycloalkyle, aryle, nitro ou carboxyle ; et
alkyle hétérocyclique et aryle hétérocyclique ont au moins un hétéroatome choisi parmi l'oxygène, l'azote et le soufre ; et
ses sels pharmaceutiquement acceptables.

2. Molécule selon la revendication 1, où :
R¹ représente cycloalkyle ou aryle ;
R² et R³ représentent indépendamment C1-C4 alkyle.

3. Molécule selon la revendication 2, où :
R⁴ est H; et
X est O.

4. Molécule selon la revendication 2, où :
R¹ représente 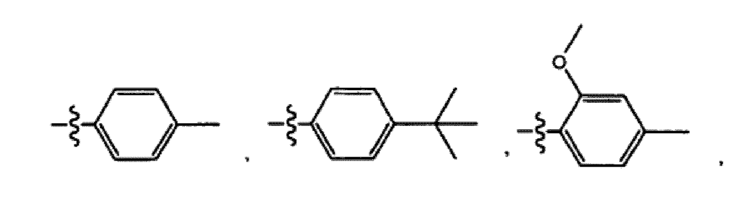 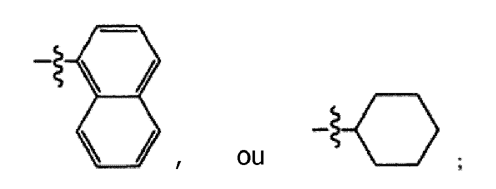
R² et R³ représentent éthyle ;
R⁴ représente H ;
R⁵ représente 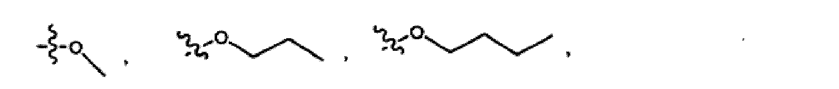 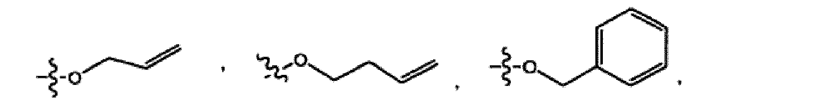 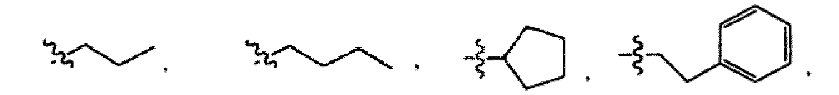 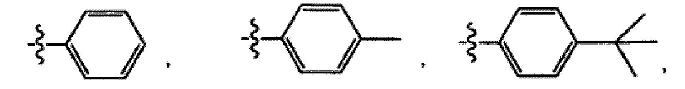 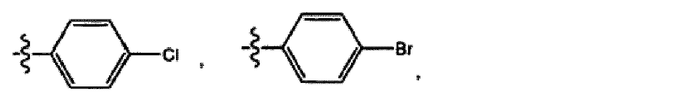 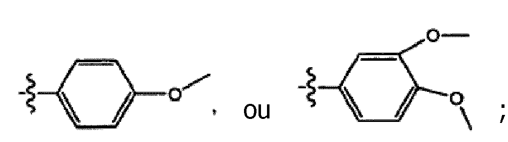 et
X représente O.

5. Molécule selon la revendication 2, où :
R¹ représente 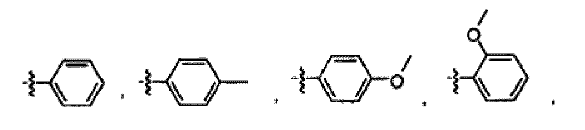 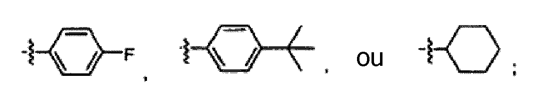
R² et R³ représentent éthyle ;
R⁴ représente H ;
R⁵ représente 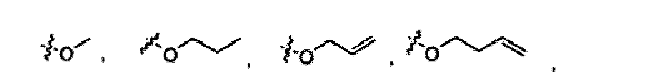 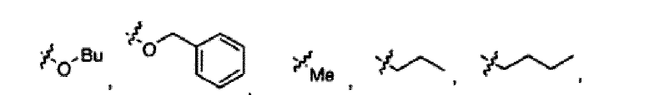 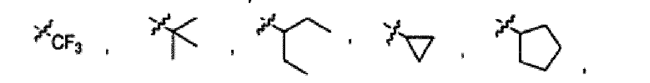 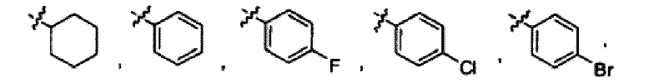 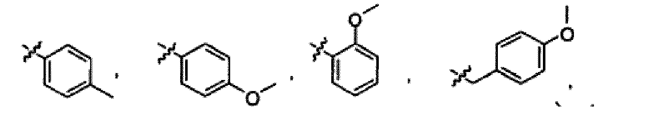 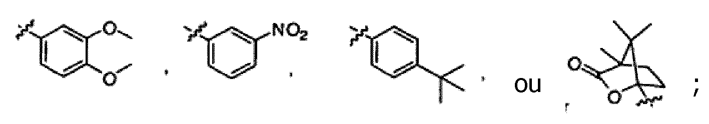 et
X représente O.

6. Molécule selon la revendication 5, où :
(i) R¹ représente 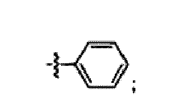 et
R⁵ représente 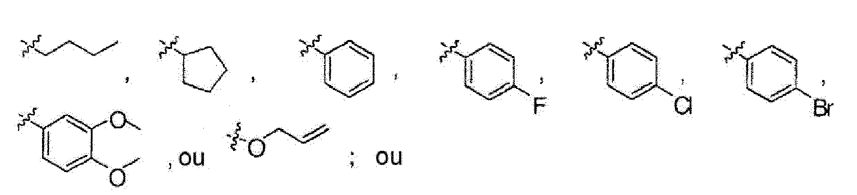
(ii) R¹ représente 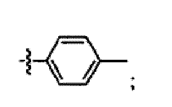 et
R⁵ représente 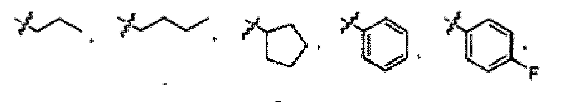 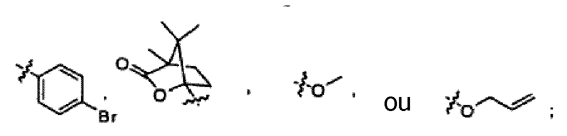 ou
(iii) R¹ représente 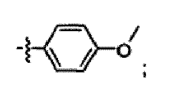 et
R⁵ représente 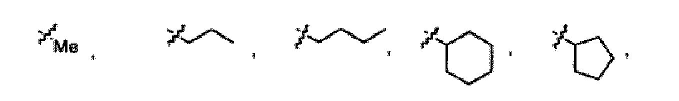 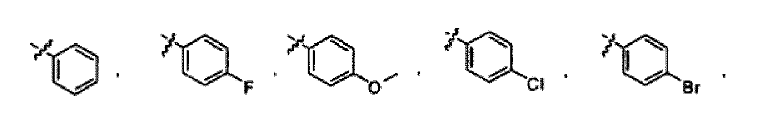 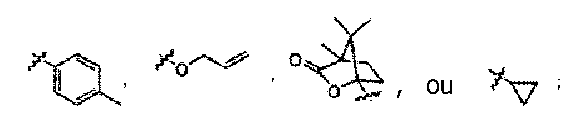 ou
(iv) R¹ représente 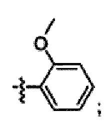 et
R⁵ représente 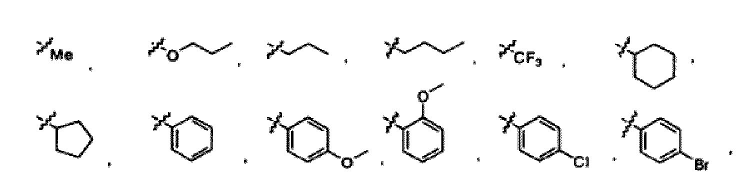 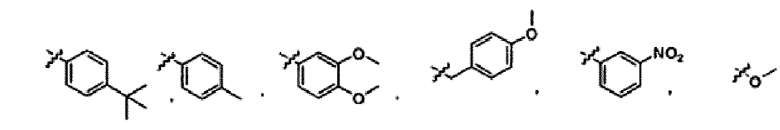 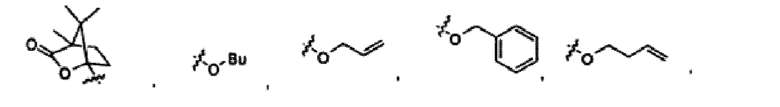 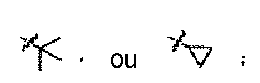 ou
(v) R¹ représente 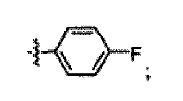 et
R⁵ représente 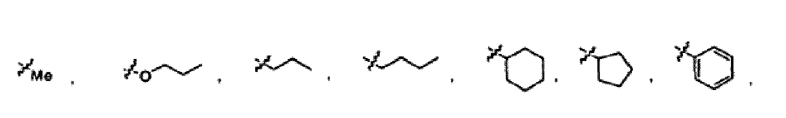 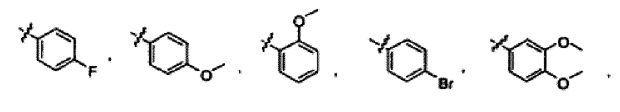 ou 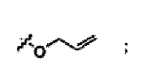 ou
(vi) R¹ représente 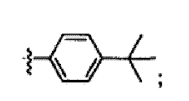 et
R⁵ représente 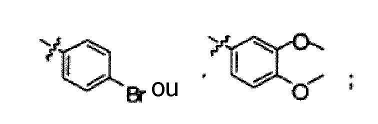 ou
(vii) R¹ représente 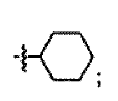 et
R⁵ représente

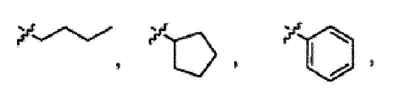

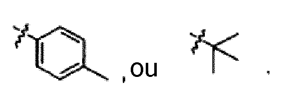

7. Molécule selon la revendication 1, où :
quand R² représente H, R³ n'est pas méthyle.

8. Composé de formule I ou ses sels pharmaceutiquement acceptables tels que définis dans la revendication 1 destinés à être utilisés dans le traitement d'un patient infecté avec *Mycobacterium tuberculosis* I.

9. Composé de formule I ou ses sels pharmaceutiquement acceptables tels que définis dans la revendication 1 destinés à être utilisés dans le traitement d'un patient infecté avec *Francisella tulerensis.*

10. Composé ou sel pharmaceutiquement acceptable selon la revendication 1 ou destiné à être utilisé selon l'une quelconque des revendications 8 ou 9, où :
R⁶, R⁷, R⁸, R⁹ et R¹⁰ représentent indépendamment alkyle, cycloalkyle ou aryle.

11. Molécule ayant la formule I 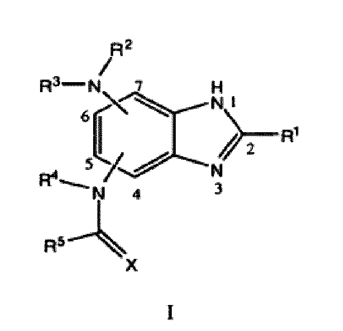 où :
R¹ représente NH₂, NHR⁶, NR⁹R¹⁰, NR⁶CONR⁹R¹⁰, NR⁶CSNR⁹R¹⁰, OH, OR⁶, SH, SR⁶, CHO, COOR⁶, COR⁶, CH₂OH, CR⁷R⁸OH, CH₂OR⁶, CR⁷R⁸OR⁶, CH₂NH₂, CR⁷R⁸NH₂, CR⁷R⁸NR⁹R¹⁰, alkyle, cycloalkyle, aryle ou halo ;
R² et R⁴ représentent indépendamment H, alkyle, cycloalkyle ou aryle ;
R⁵ représente H, R⁶, OR⁶, SR⁶, NH₂, NHR⁶ ou NR⁹R¹⁰ ;
R⁶, R⁷, R⁸, R⁹ et R¹⁰ représentent indépendamment alkyle, cycloalkyle ou aryle ;
R² et R³ ; R⁴ et R⁵ ; et R⁹ et R¹⁰ indépendamment, peuvent être combinés pour représenter un alkyle hétérocyclique ou un aryle hétérocyclique ;
R⁷ et R⁸ peuvent être combinés pour représenter un cycloalkyle ;
et où :
X représente O ; et
R³ représente alkyle, cycloalkyle, aryle, CO(cycloalkyle) ou CO(cycloaryle) ;
ou bien
X représente S, NH ou NR⁶ ; et
R³ représente alkyle, cycloalkyle, aryle ou COR⁶ ;
ou bien
X représente O ;
R³ représente COCH₃ ; et
R¹, R², R⁴, R⁵ et R⁶ sont tels que représentés dans les composés de formule I ci-dessous : 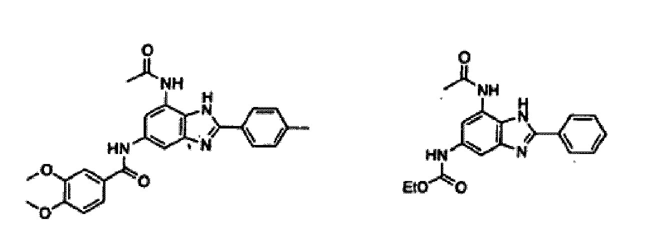 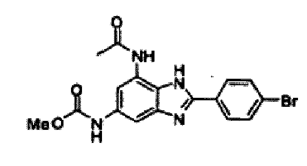 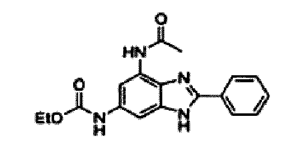 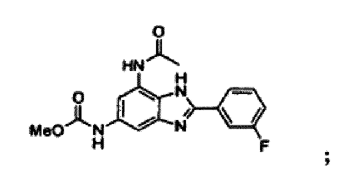
et où :
les groupes alkyle sont ramifiés ou non ramifiés, saturés ou insaturés et ont 1-18 atomes de carbone dans leur chaîne la plus longue ;
les groupes cycloalkyle sont des systèmes cycliques, carbocycliques ou hétérocycliques, condensés ou non condensés, non aromatiques ayant un total de 5-16 chaînons cycliques incluant les cycles substituants ;
les groupes aryle sont carbocycliques ou hétérocycliques ;
les groupes aryle carbocycliques sont des systèmes cycliques condensés ou non condensés ayant un total de 6-16 chaînons cycliques incluant les cycles substituants ;
les groupes aryle hétérocycliques sont des systèmes cycliques condensés ou non condensés ayant un total de 5-16 chaînons cycliques incluant les cycles substituants ;
les substituants halo sont fluoro, chloro ou bromo ;
chaque alkyle, cycloalkyle et aryle, indépendamment, peut être non substitué ou substitué avec un ou plusieurs substituants à une position quelconque ;
les substituants d'alkyle sont halo, hydroxyle, OR⁶, SR⁶, NH₂, NHR⁶, NR⁹R¹⁰, cycloalkyle ou aryle ;
les substituants de cycloalkyle sont halo, hydroxyle, OR⁶, SR⁶, NH₂, NHR⁶, NR⁹R¹⁰, alkyle, cycloalkyle ou aryle ;
les substituants d'aryle sont halo, hydroxyle, OR⁶, SR⁶, NH₂, NHR⁶, NR⁹R¹⁰, alkyle, cycloalkyle, aryle, nitro ou carboxyle ; et
alkyle hétérocyclique et aryle hétérocyclique ont au moins un hétéroatome choisi parmi l'oxygène, l'azote et le soufre ; et
ses sels pharmaceutiquement acceptables.

12. Composé de formule I ou ses sels pharmaceutiquement acceptables tels que définis dans la revendication 11 destinés à être utilisés dans le traitement d'un patient infecté avec *Mycobacterium tuberculosis.*

13. Composé de formule I ou ses sels pharmaceutiquement acceptables tels que définis dans la revendication 11 destinés à être utilisés dans le traitement d'un patient infecté avec *Francisella tulerensis.*
